# EUROPEAN PATENT SPECIFICATION

(11) **EP 1 129 219 B1**
(45) Date of publication and mention of the grant of the patent: **18.08.2004**
(21) Application number: 99971866.1
(22) Date of filing: 09.11.1999
(51) Int. Cl.: C12Q 1/68

(54) **RESTRICTED AMPLICON ANALYSIS**
ANALYSE VON "AMPLIKONS" ERHALTEN DURCH RESTRIKTIONSENDONUKLEASEN
ANALYSE D'AMPLICON RESTREINT

(30) Priority: 09.11.1998 US 107293 P
(43) Date of publication of application: 05.09.2001
(73) Proprietor: Methexis Genomics N.V., 9052 Zwijnaade (BE)
(72) Inventor: ZABEAU, Marc, B-9000 Gent (BE); STANSSENS, Patrick, B-9810 Nazareth (BE)
(74) Representative: De Clercq, Ann
(86) International application number: PCT/IB1999/001958
(87) International publication number: WO 2000/028081

(56) References cited:
- EP-A- 0 534 858
- WO-A-91/17262
- WO-A-96/17082
- WO-A-97/29211
- WO-A-97/34015
- WO-A-98/12352
- US-A- 5 741 678
- DATABASE BIOSIS [Online] BIOSCIENCES INFORMATION SERVICE, PHILADELPHIA, PA, US1998 DU YANG-ZHU ET AL: "Multiple exon screening using restriction endonuclease fingerprinting (REF): Detection of six novel mutations in the L1 cell adhesion molecule (L1CAM) gene." Database accession no. PREV199800180156 XP002139122 & HUMAN MUTATION 1998, vol. 11, no. 3, 1998, pages 222-230, ISSN: 1059-7794
- DATABASE BIOSIS [Online] BIOSCIENCES INFORMATION SERVICE, PHILADELPHIA, PA, US1998 GILAD SHLOMIT ET AL: "Identification of ATM mutations using extended RT-PCR and restriction endonuclease fingerprinting, and elucidation of the repertoire of A-T mutations in Israel." Database accession no. PREV199800093427 XP002139123 & HUMAN MUTATION 1998, vol. 11, no. 1, 1998, pages 69-75, ISSN: 1059-7794
- VOS P ET AL: "AFLP: a new technique for DNA fingerprinting" NUCLEIC ACIDS RESEARCH,GB,OXFORD UNIVERSITY PRESS, SURREY, vol. 23, no. 21, 1995, pages 4407-4414-4414, XP002109691 ISSN: 0305-1048 cited in the application
- WANG D G ET AL: "Large-scale identification, mapping, and genotyping of single-nucleotide polymorphisms in the human genome" SCIENCE,US,AMERICAN ASSOCIATION FOR THE ADVANCEMENT OF SCIENCE,, vol. 280, 1 January 1998 (1998-01-01), pages 1077-1082, XP002089398 ISSN: 0036-8075 cited in the application

### SEQUENCE LISTING

<110> METHEXIS N.V.
<120> RESTRICTED AMPLICON ANALYSIS
<130> 29314/34158A
<140>
   <141>
<150> 60/107,293
   <151> 1998-11-09
<160> 28
<170> PatentIn Ver. 2.0
<210> 1
   <211> 17
   <212> DNA
   <213> Artificial Sequence
<220>
   <223> Description of Artificial Sequence: primer
<400> 1
   ctcgtagact gcgtacc 17
<210> 2
   <211> 18
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 2
   aattggtacg cagtctac 18
<210> 3
   <211> 16
   <212> DNA
   <213> Artificial Sequence
<220>
   <223> Description of Artificial Sequence: primer
<400> 3
   gacgatgagt cctgag 16
<210> 4
   <211> 14
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 4
   tactcaggac tcat 14
<210> 5
   <211> 18
   <212> DNA
   <213> Artificial Sequence
<220>
   <223> Description of Artificial Sequence: primer
<220>
   <221> misc_feature
   <222> (17)
   <223> At position 17 N = A, C, G, or T
<220>
   <221> misc_feature
   <222> (18)
   <223> At position 18 N = A, C, G, or T
<400> 5
   gactgcgtac caattcnn 18
<210> 6
   <211> 19
   <212> DNA
   <213> Artificial Sequence
<220>
   <223> Description of Artificial Sequence: primer
<220>
   <221> misc_feature
   <222> (17)
   <223> At position 17 N = A, C, G, or T
<220>
   <221> misc_feature
   <222> (18)
   <223> At position 18 N = A, C, G, or T
<220>
   <221> misc_feature
   <222> (19)
   <223> At position 19 N = A, C, G, or T
<400> 6
   gatgagtcct gagtagnnn 19
<210> 7
   <211> 21
   <212> DNA
   <213> Artificial Sequence
<220>
   <223> Description of Artificial Sequence: primer
<400> 7
   ctcgtagact gcgtacatgc a 21
<210> 8
   <211> 14
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 8
   tgtacgcagt ctac 14
<210> 9
   <211> 16
   <212> DNA
   <213> Artificial Sequence
<220>
   <223> Description of Artificial Sequence: primer
<400> 9
   gactgcgtac atgcag 16
<210> 10
   <211> 16
   <212> DNA
   <213> Artificial Sequence
<220>
   <223> Description of Artificial Sequence: primer
<400> 10
   gatgagtcct gagtag 16
<210> 11
   <211> 21
   <212> DNA
   <213> Artificial Sequence
<220>
   <223> Description of Artificial Sequence:.primer
<400> 11
   gagcatctga cgcatgttgc a 21
<210> 12
   <211> 14
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 12
   acatgcgtca gatg 14
<210> 13
   <211> 16
   <212> DNA
   <213> Artificial Sequence
<220>
   <223> Description of Artificial Sequence: primer
<400> 13
   ctgctactca ggactg 16
<210> 14
   <211> 14
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 14
   tacagtcctg agta 14
<210> 15
   <211> 16
   <212> DNA
   <213> Artificial Sequence
<220>
   <223> Description of Artificial Sequence: primer
<400> 15
   ctgacgcatg ttgcag 16
<210> 16
   <211> 16
   <212> DNA
   <213> Artificial Sequence
<220>
   <223> Description of Artificial Sequence: primer
<400> 16
   ctactcagga ctgtag 16
<210> 17
   <211> 20
   <212> DNA
   <213> Artificial Sequence
<220>
   <223> Description of Artificial Sequence: primer
<400> 17
   ctcgtagact gcgtacccat 20
<210> 18
   <211> 15
   <212> DNA
   <213> Artificial Sequence
<220>
   <223> Description of Artificial Sequence: primer
<220>
   <223> As presented in the Sequence Listing the nucleotide sequence reads in the 5' to 3' direction. As presented in the specification the sequence reads in the 3' to 5' direction.
<400> 18
   gggtacgcag tctac 15
<210> 19
   <211> 16
   <212> DNA
   <213> Artificial Sequence
<220>
   <223> Description of Artificial Sequence: primer
<400> 19
   gactgcgtac ccatta 16
<210> 20
   <211> 20
   <212> DNA
   <213> Artificial Sequence
<220>
   <223> Description of Artificial Sequence: primer
<400> 20
   gagcatctga cgcatgggat 20
<210> 21
   <211> 15
   <212> DNA
   <213> Artificial Sequence
<220>
   <223> As presented in the Sequence Listing the nucleotide sequence reads in the 5' to 3' direction. As presented in the specification the sequence reads in the 3' to 5' direction.
<220>
   <223> Description of Artificial Sequence: primer
<400> 21
   cccatgcgtc agatg 15
<210> 22
   <211> 16
   <212> DNA
   <213> Artificial Sequence
<220>
   <223> Description of Artificial Sequence: primer
<400> 22
   ctgacgcatg ggatta 16
<210> 23
   <211> 16
   <212> DNA
   <213> Artificial Sequence
<220>
   <223> Description of Artificial Sequence: primer
<400> 23
   gtcctcatcg agcatg 16
<210> 24
   <211> 14
   <212> DNA
   <213> Artificial Sequence
<220>
   <223> As presented in the Sequence Listing the nucleotide sequence reads in the 5' to 3' direction. As presented in the specification the sequence reads in the 3' to 5' direction.
<220>
   <223> Description of Artificial Sequence: primer
<400> 24
   cgcatgctcg atga 14
<210> 25
   <211> 16
   <212> DNA
   <213> Artificial Sequence
<220>
   <223> Description of Artificial Sequence: primer
<400> 25
   cctcatcgag catgcg 16
<210> 26
   <211> 17
   <212> DNA
   <213> Artificial Sequence
<220>
   <223> Description of Artificial Sequence: primer
<400> 26
   gagcatctga cgcatcc 17
<210> 27
   <211> 18
   <212> DNA
   <213> Artificial Sequence
<220>
   <223> As presented in the Sequence Listing the nucleotide sequence reads in the 5' to 3' direction. As presented in the specification the sequence reads in the 3' to 5' direction.
<220>
   <223> Description of Artificial Sequence: primer
<400> 27
   aattggatgc gtcagatg 18
<210> 28
   <211> 16
   <212> DNA
   <213> Artificial Sequence
<220>
   <223> Description of Artificial Sequence: primer
<400> 28
   ctgacgcatc caattc 16

## Description

### Field of the Invention

The present invention generally provides a method which facilitates the detection of polymorphisms (or mutations). The method is directed to the analysis of so-called endonuclease site polymorphisms (ESPs) that result in the gain or loss of a restriction endonuclease site. In essence, the ESP is probed with the restriction endonuclease reagent prior to amplification, whereby amplification is prevented and consequently no signal is observed when cleavage takes place. Unambiguous allele calling is performed by comparing the signals obtained with and without cleavage with the restriction endonuclease reagent. The method is particularly useful for multiplex genotyping, involving the parallel analysis of large numbers of single nucleotide polymorphisms. Preferred methods for detecting the amplicons involve hybridization to an arrayed or otherwise identifiable set of cognate probe fragments or oligonucleotides.

### Background of the Invention

Molecular approaches for genetic analyses trace the nucleotide sequence variation that occurs naturally and randomly in the genomes of all living species. Knowledge of the DNA polymorphisms among individuals and between populations is important in understanding the complex links between genotypic and phenotypic variation. In the absence of complete data about sequence variation, one relies on the ability to identify 'nearby' markers that allow to infer the location of certain relevant loci or causal sequence variations. The informativeness of the marker depends on the magnitude of the linkage disequilibrium. Markers can be used in linkage studies to search for candidate genes and in association studies to identify the functional allelic variation on candidate genes that influence inter-individual variation.

The vast majority of sequence variation consists of nucleotide substitutions, often referred to as single nucleotide polymorphism's (SNPs), resulting from mutations that have accumulated during evolution. Most of these nucleotide changes are genetically silent; i.e., they have no measurable biological effect, but provide an immense reservoir of variation in DNA structure. Most methods for genetic analysis used today rely on the detection of nucleotide sequence variation which can be measured by DNA fragment analysis using electrophoretic separation, in which DNA fragments are fractionated based on size or conformation. Occasionally the nucleotide sequence variation will affect either the presence of the DNA fragment or its mobility. In this way the primary nucleotide sequence variation will give rise to easily detectable DNA fragment polymorphism. Since polymorphic DNA fragments are derived from precise locations on the organism's genome, they can serve as reliable genetic markers, or landmarks to identify and locate genes.

A host of assays to detect DNA polymorphisms, and SNPs in particular, have been developed. In some of these assays (*e.g.,* RFLP [Botstein, D., White, R.L., Skolnich, M., Davis, R.W., *Am. J. Hum. Genet.* **32**:314-331 (1998)], CAPS [Konieczny, A. Ausubel, J.F., *Plant J.* **4**:403-410 (1993)], dCAPS [Neff, M.M. Neff, J.D., Chory, J., Pepper, A.E., *The Plant Journal* **14**:387-392 (1998)], PIRA [Steinborn, R., Muller, M., Brem, G., *Biochim. Biophys. Acta* **1397**:295-304 (1998)]), restriction enzymes are used to detect polymorphic nucleotide sequences that affect cleavage. The specificity of restriction enzymes is such that they exhibit a unique sensitivity to detect single nucleotide differences occurring in their recognition sites. The principal strengths of restriction enzyme-based genetic analyses are the ease of use and the robustness of the assays. In the majority of the cases, the restriction site polymorphism is used to detect known, previously identified SNPs and the assay consists of any electrophoretical fragment analysis. In one report, the allelic variation is detected in a solid-phase ELISA-type setting [Truett, G.E., Walker, J.A., Wilson, J.B., Redmann, S.M. Jr., Tulley, R.T., Eckardt, G.R., Plastow, G., *Mamm. Genome* **9**:629-632 (1998)].

In WO 91/17269, Lerner *et al.* describe a different method for mapping a eukaryotic chromosome by restriction endonuclease mapping of discrete DNA sequences which are complementary to a region of a eukaryotic chromosome.

Vos *et al., Nucl. Acids Res.* **23**:4407-4414 (1995) and EP 0 534 858 describe a technique for DNA fingerprinting called AFLP which is based on the selective polymerase chain reaction based application of restriction fragments of a digest of genomic DNA. The application reaction depends on the use of primers that extend into restriction fragments amplifying only those fragments in which prior extensions match the nucleotide sequence flanking the restriction sites.

Another method utilizing DNA amplification steps is set out in Williams *et al., Nucl. Acids Res.* **18**:6531-6535 (1990), whd describe a DNA fingerprinting method termed random amplified polymorphic DNA.

DNA amplification fingerprinting was described by Caetano Ariolles in *Bio*/*Technology* **9**:553-557 (1-991). Still another fingerprinting technique called arbitrarily primed PCR was described in Welsh *et al., Nucl. Acids Res.* **18**:7213-7218 (1990) and Welsh *et al., Nucl. Acids Res.* **19**:861-866 (1991).

In WO 94/11530, Cantor *et al.* describe materials and methods for position and sequencing by hybridization. Cantor *et al.* also describe methods for creating assays of DNA probes useful in the practice of their method.

The major shortcoming of the current methods of genetic analysis is the limited resolution of the DNA fragment analysis systems, namely the number of DNA fragments that can be separated in a single assay. Generally the fractionation resolution ranges from tens to a couple of hundred DNA fragments, at the most. Consequently, current genetic analysis methods are limited to a few hundred to a thousand genetic markers. While this resolution has been sufficient for analyzing simple genetic traits determined by single genes, the analysis of complex traits, which is now being undertaken and which involve general or many different genes, will require the analysis of a much larger number of genetic markers. It is anticipated that such studies will require from a few thousand to possibly several hundred thousand genetic markers. Although this could conceivably be accomplished by performing many parallel assays, such scaling up will be cost- and labor prohibitive.

For example in WO97/34015, WO98/12352, WO91/17262 and US5,741,678 methods are described to analyse SNPs in genomes. It is clear from these applications and this patent that no complex genotyping could be performed using said prior art techniques.

A technology that has great potential and which is generating widespread interest in the so-called micro-array technology (DNA chips). In general, these methods are based on measurement of the hybridization of DNA sequences in solution to probe sequences that are arrayed on a solid surface. When assaying nucleotide polymorphisms, the detector relies on the small differences in hybridization efficiency between two different DNA sequences. In one format, fluorescently labeled sample DNA is hybridized to dense arrays of probe nucleic acids, sequence-specific hybridization signal is detected by scanning confocal microscopy, and DNA variants scored as (predictable) differences in the hybridization pattern. The micro-arrays are fabricated either by in-situ light-directed oligonucleotide synthesis [Fodor, S.P.A. *et al., Science* **251**: 767 (1991)] or by spotting DNA (off chip synthesized oligonucleotides or PCR fragments) in an automated procedure. The technology has already been demonstrated in the scoring of mutations in mitochondrial DNA [Chee, M. *et al., Science* **274**: 610-614 (1996)], the HIV genome [Lipshutz, R.J. *et al.,* , *Biotechniques* **19**: 442-447 (1995)], the CFTR cystic fibrosis gene [Cronin, M.T. *et aL, Human Mut.***7**: 244-255 (1996)], the BRCA1 breast cancer gene (Hacia, G.H. *et al., Nat.* Genet. **14**: 441-447 (1996)] as well as the entire yeast genome [Winzeler, E.A. *et al., Science* **281**:1194 (1998)]. In comparison with most other assays, micro-arrays provide a platform for high-throughput, massively parallel polymorphism detection.

A major disadvantage with the use of microarrays relates to the complexity of the hybridization reaction. The detection relies on the very small difference in hybridization of DNA sequences differing by only one nucleotide. In general, a set of 4 oligonucleotides, differing only in the identity of the central base, is synthesized for each position in the target sequence that has to be interrogated. In practice, the number of oligonucleotides needed to correctly genotype one SNP is much larger, involving up to 56 different oligonucleotides spanning the variable base [Wang *et al., Science* **280**: 1077-1082 (1998)). The degree of redundancy is also dramatic if one wants to screen the target DNA for all possible mutations; the design then includes overlapping oligonucleotide-sets that are offset by one base (a process known as tiling). It should be noted that the detection of SNPs by hybridization to arrays depends on the use of short oligonucleotide probes. With longer probes such as DNA fragments in the size range of 50 to 500 base pairs or larger, it is not possible to distinguish the SNP alleles.

### Summary of the Invention

The present invention is directed to methods for genotyping polymorphisms that result in the gain or loss of an endonuclease cleavage site. Such polymorphisms are referred to hereinafter as endonuclease site polymorphisms (ESPs). Polymorphisms detectable according to the methods of the present invention include single nucleotide polymorphisms (SNPs).

The present invention relates to a method to perform multiplex genotyping whereby multiple endonuclease site polymorphisms (ESPs) are simultaneously analyzed in sample DNA, the method comprising:
(a) isolating sample DNA;
(b) treating the sample DNA of step (a) with one or more sampling restriction endonuclease reagent(s) to derive a set of concomitantly amplifiable sample restriction endonuclease reagent treated target DNA fragments,
(c) treating the target DNA fragments obtained in step (b) with a restriction reagent, the probe restriction endonuclease reagent; with said probe restriction endonuclease reagent being different from the sampling restriction endonuclease reagent(s) of step (b) to probe the allelic state at specific ESP-sites,
(d) amplifying selectively or non-selectively the probe restriction endonuclease reagent treated target DNA fragments of step (c) in one or more multiplex reactions;
(e) analyzing the DNA of step (d) by hybridization to an array containing spatially addressable probes or oligonucleotides or a multitude of identifiable solid phase particles each carrying a different probe or oligonucleotide to determine which target fragments are amplified and/or which target fragments are not amplified; and wherein target DNA fragments which are amplified in step (d) lack a recognition site for the probe restriction endonuclease reagent and target fragments having a recognition site for the probe restriction endonuclease reagent are not amplified, whereby each cognate probe fragment or oligonucleotide allows to characterize one or more specific ESP(s).

The present invention further relates to the above-mentioned method wherein the order of the steps (b) and (c) are reversed or carried out simultaneously.

The present invention also relates to said method wherein said concomitantly amplifiable DNA fragments of step (b) are derived from sample DNA by treatment of the sample DNA with a first and a second sampling restriction endonuclease reagent.

Further, the present invention relates to said method wherein said first sampling restriction endonuclease reagent has a recognition sequence of six or more nucleotides and said second sampling restriction endonuclease reagent has a recognition sequence of four or fewer nucleotides.

The present invention also relates to the above-mentioned method wherein said concomitantly amplifiable target DNA fragments are derived by stepwise treatment of said sample DNA with the first and the second sampling restriction endonuclease reagents.

Further, the present invention relates to said method wherein said concomitantly amplifiable DNA fragments of step (b) are modified by ligation of adapters to both termini of said fragments.

The present invention also relates to said method wherein the DNA fragments of step (c) are pre-amplified and used as template for the amplification of step (d).

The present invention also relates to said method wherein the pre-amplification is performed using primers recognizing the adapters, ligated to the termini of all or a subset of the sampling restriction endonuclease treated DNA fragments.

Further the present invention relates to said method wherein in step (d) the DNA fragments are amplified selectively in one or more multiplex reactions using dedicated primers that flank said ESPs, or, using primers that recognize the adapters, ligated to the termini of the sampling restriction endonuclease treated DNA fragments, and wherein the selective amplifications are performed with EcoRI and BfaI primers having two and three selective nucleotides recognizing the target DNA of step (b), respectively.

The present invention also relates to the above-mentioned method wherein in step (d) the DNA fragments are amplified non-selectively in a multiplex reaction using primers recognizing the adapters ligated to the termini of the sampling restriction endonuclease treated DNA fragments.

Further, the present invention relates to a method to perform multiplex genotyping whereby multiple known endonuclease site polymorphisms (ESPs) are simultaneously analyzed in sample DNA, the method comprising:
(a) isolating sample DNA;
(b) treating the sample DNA obtained in step (a) with one or more probe restriction endonuclease reagent(s) resulting in the production of probe restriction endonuclease reagent treated target DNA fragments, to probe the allelic state at specific ESP-sites,
(c) concomitantly and selectively amplifying multiple target DNA fragments having an endonuclease site polymorphism (ESP) for the probe restriction endonuclease in one or more multiplex reactions using dedicated primers that flank said ESPs;
(d) analyzing the DNA of step (c) by hybridization to an array containing spatially addressable probes or oligonucleotides or a multitude of identifiable solid phase particles each carrying a different probe or oligonucleotide to determine which target fragments are amplified and/or which target fragments are not amplified; and wherein target DNA fragments which are amplified lack a recognition site for the probe restriction endonuclease reagent and target fragments . having a recognition site for the probe restriction endonuclease reagent are not amplified in step (c), whereby each cognate probe fragment or oligonucleotide allows to characterize one or more specific ESP(s).

The present invention further relates to said method wherein said probe restriction endonuclease reagent has a recognition sequence comprising six or more nucleotides.

The present invention also relates to said method wherein said probe restriction endonuclease reagent has a recognition sequence comprising four or more nucleotides.

Further, the present invention relates to said method wherein the said probe restriction endonuclease has a recognition sequence of two nucleotides.

The present invention also relates to said method wherein said endonuclease site polymorphism (ESP) is an alteration in the nucleotide sequence of a concomitantly amplifiable target fragment giving rise to a nucleotide sequence that is recognized and cut by the probe restriction endonuclease reagent.

Further the present invention relates to said method wherein said endonuclease site polymorphism (ESP) is an alteration in the nucleotide sequence, of a concomitantly amplifiable target fragment which eliminates a recognition sequence for said probe restriction endonuclease reagent.

The invention further relates to the above-mentioned method wherein said amplification is carried out by a polymerase chain reaction.

The present invention also relates to said method wherein said probes for hybridization are attached to a solid support which is selected from a group consisting of a planar solid support, a bead, a sphere and a polyhedron.

The invention further relates to said method wherein said DNA analyzed by hybridization consists of a mixture of two differentially labeled counterparts wherein one part is derived from the sample material with omission of the digestion with probe restriction endonuclease reagent.

The present inventions also relates to said method wherein said hybridization probes are fragments derived from the sample DNA and having an endonuclease site polymorphism (ESP) for the probe restriction endonuclease.

Finally, the present invention relates to said method wherein said hybridization probes are synthetic oligonucleotides based on the sequence of concomitantly amplifiable DNA fragments having an endonuclease site polymorphism (ESP) for the probe restriction endonuclease.

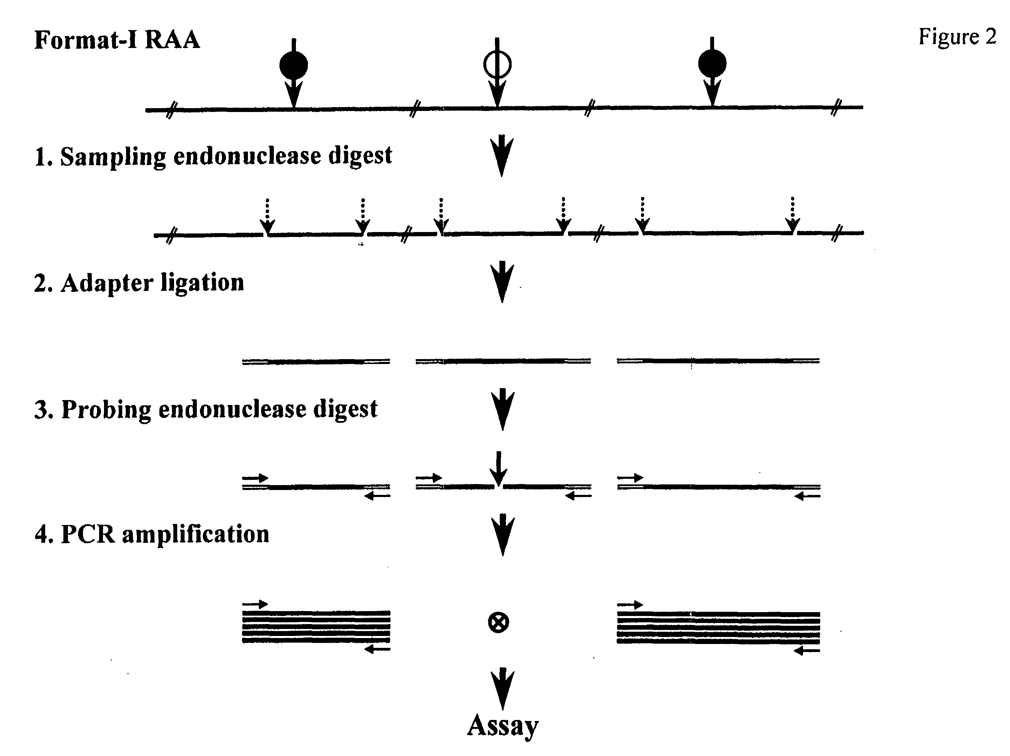

In one aspect, the present invention is directed to RAA-methods, which comprise the preparation of concomitantly amplifiable DNA segments by digestion of the starting DNA with one or more restriction endonucleases, collectively referred to herein as sampling enzymes. This method is herein referred to as format-I RAA and is diagrammed in Figure 2. The digested starting DNA may be further modified at its termini by the addition of adapters, which may serve to prime an amplification reaction (see Figure 2). Once sample DNA is obtained, it is treated with a different restriction enzyme, the probing enzyme also referred to as a probe restriction endonuclease substantial fraction of the sample fragments contain a single recognition site for the probe endonuclease reagent. In general, probe enzymes used with format-I RAA preferably have as a recognition site a nucleotide sequence of less than six nucleotides.

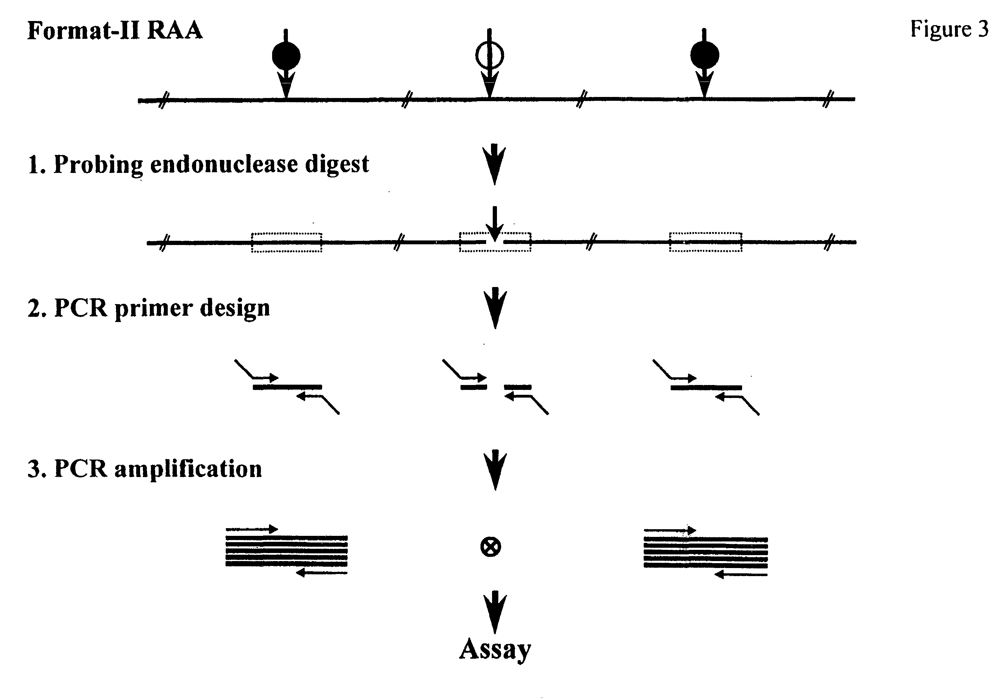

In another aspect, the present invention is directed to methods for format-II RAA for the detection of ESPs, as diagrammed in Figure 3. Format-II RAA operates on the same principal as fonnat-I RAA except that the sample amplicons need not be DNA fragments, but are rather defined regions of a genome amplifiable with specific primer pairs. The amplicons of the format-II RAA are identified on the basis of sequence data; e.g. the sequence of ESP-containing restriction fragments identified using format-I RAA method or otherwise known SNPs affecting endonuclease cleavage sites. In format-II RAA, the test DNA to be analyzed is treated with a probe restriction endonuclease reagent, followed by the concomitant amplification of regions of the treated DNA (amplicons) using predetermined primers using, for example, the polymerase chain reaction as described herein. The analysis of the amplification products then proceeds as described in the format-I RAA methods described herein. As with format-I RAA, an ESP is genotyped by the presence or absence of a recognition site for the probe restriction endonuclease reagent.

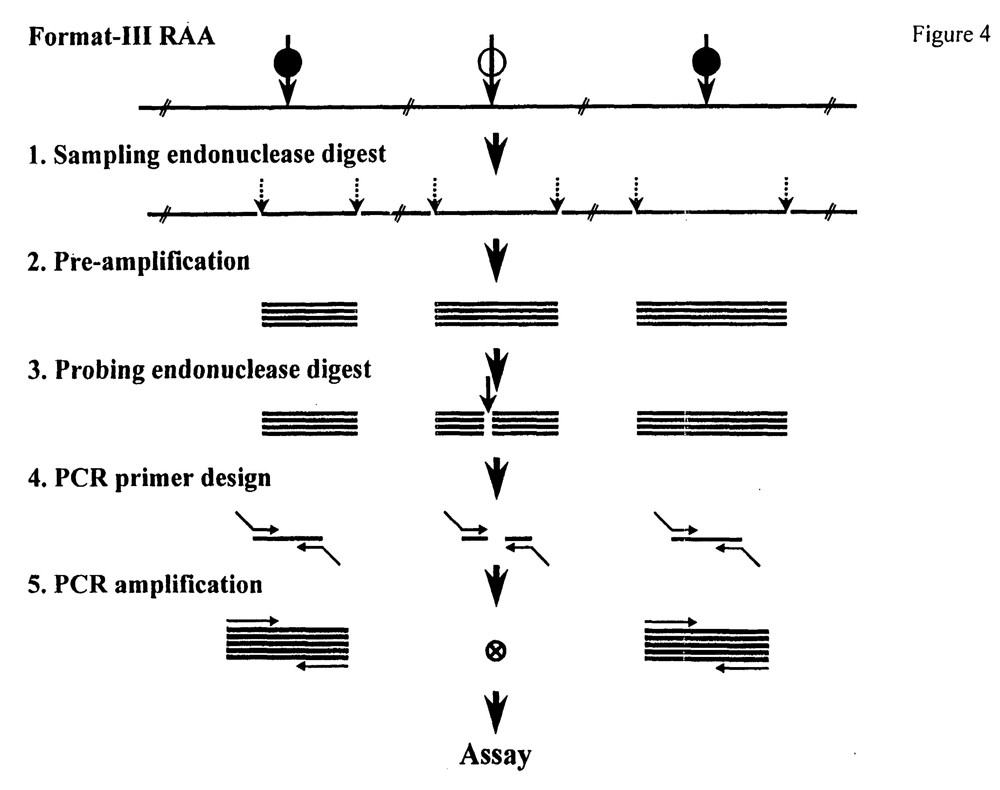

In yet another aspect, the present invention is directed to methods for format-III RAA. In essence, format-III RAA consists of a combination of the format-I and format-II approaches. One of such combinations is diagrammed in Figure 4. Test DNA, digested or not with a probe endonuclease reagent, is sampled with a pair of endonuclease reagents and the resulting fragments are co- as described in the format-I assay amplified (this step is referred to as the pre-amplification step). These pre-amplification mixtures are, in turn, used as templates for a format-II type of PCR reaction in which multiple ESP-containing regions are selectively co-amplified using specific primer sets. The analysis of the amplification products then proceeds as described before. The advantages of format-IiI RAA are that the stepwise amplification facilitates the multiplex PCR of the ESP-specific amplicons and lowers the amount of starting material required to interrogate all the ESPs.

Arrays, or microarrays of probe DNA wherein the probe DNAs are useful in the detection of ESPs are also encompassed by the present invention. Informative probe DNAs are prepared and identified as described in detail below and are then attached to a substrate for use in the hybridization reactions with concomitantly amplifiable DNA after treatment with a probe restriction endonuclease reagent and subsequent amplification.

Since the method of the invention is based on the detection of a particular kind of DNA polymorphism, which occurs in DNA of any organism, the invention will be universally applicable. The methods of the present invention may be used to genotype ESPs in a wide variety of organisms from prokaryotic organisms, such as bacteria, through complex eukaryotic organisms, viruses, or any organism having a genome however simple or complex. The methods may also be used for the analysis of extrachromosomal DNA, the DNA found in certain cellular organelles, cDNA preparations, or DNA libraries, such as yeast artificial chromosome libraries and others. Furthermore, based on the large body of DNA sequence data at hand, it is predicted that the genomes of higher organisms carry several hundreds of thousands of such DNA polymorphism. Consequently, the new method is capable of diagnosing the immense number of genetic markers that are needed to unravel complex traits. The method is of tremendous value for high throughput genetic analysis in the emerging field of pharmacogenomics. Similarly, the method has great potential in the field of animal and plant breeding, where high resolution genetic analysis will be needed to identify the genes involved in quantitative agronomic traits.

Various aspects of the present invention are described in more detail below *(see* Detailed Description of the Invention). Variations in each of these aspects will be readily appreciated by one of ordinary skill in the art and one with the scope of the invention.

### Brief Description of the Drawings

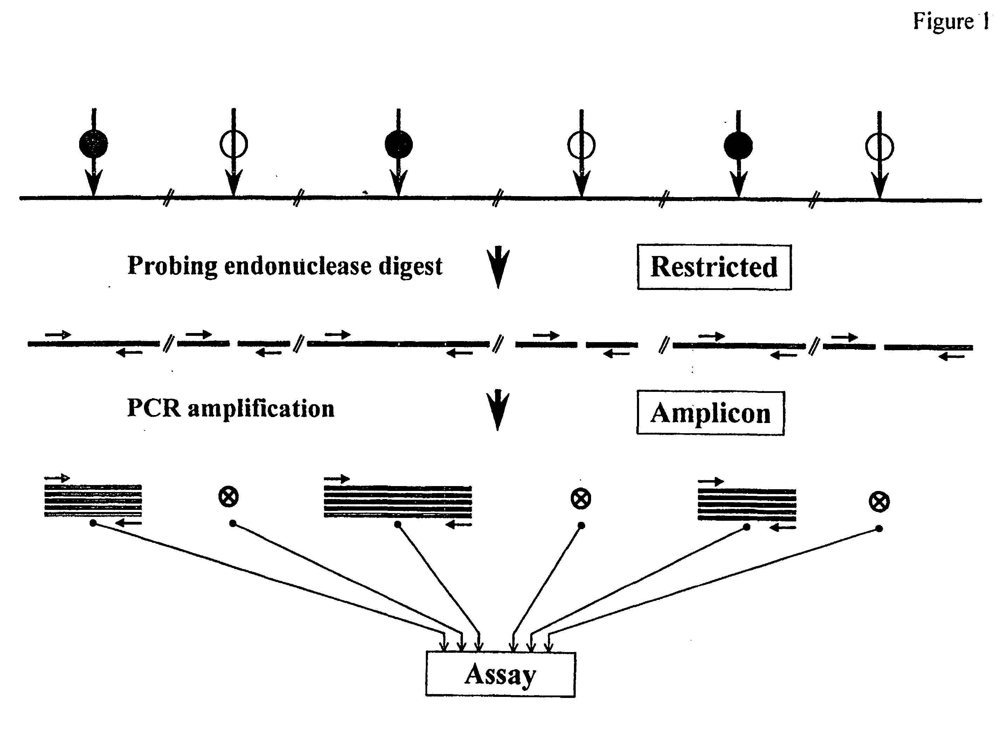

Figure 1 depicts the general concept of the Restricted Amplicon Assay. The vertical arrows indicate the positions of the ESPs. The open circles denote the probing enzyme sites that are present, while the closed circles denote the mutated sites. The first step involves cleavage of the test DNA with the probing endonuclease. The second step involves PCR amplification of DNA segments comprising the ESPs. The small horizontal arrows denote the PCR primers flanking the ESPs. When cleavage occurs the DNA is cut between the PCR primers, preventing the subsequent amplification of the DNA segment comprising those ESPs. Only those DNA segments that were not cleaved are amplified. The final step comprises assaying the amplicons.

Figure 2: Diagrammed representation of fonnat-I RAA. The vertical arrows indicate the positions of the ESPs, with the open and closed circles denoting the probing enzyme sites that are respectively present and absent. Step 1 represents the sampling enzyme cleavage step. The vertical dotted arrows indicate the positions of the sampling enzyme cleavage sites. Step 2 represents the adapter ligation step. The open lines represent the adapters ligated to the ends of the sampled restriction fragments. Step 3 represents the probing enzyme cleavage step and the small horizontal arrows denote the PCR primers matching the adapter sequences. Step 4 represents the PCR amplification step in which only the sample fragments that are not cleaved by the probing enzyme are amplified. The crossed circles represent the fragments that are not amplified.

Figure 3: Diagrammed representation of format-II RAA. The vertical arrows indicate the positions of the ESPs, with the open and closed circles denoting the probing enzyme sites that are respectively present and absent. Step 1 represents the probing enzyme cleavage step. The dotted boxes denote the DNA sequences flanking the ESP sites. Step 2 represents the PCR primer design. The small horizontal arrows denote the PCR primers flanking the ESPs Step 3 represents the PCR amplification step in which only the sample fragments that are not cleaved by the probing enzyme are amplified. The crossed circles represent the fragments that are not amplified.

Figure 4: Diagrammed representation of format-III RAA. The vertical arrows indicate the positions of the ESPs, with the open and closed circles denoting the probing enzyme sites that are respectively present and absent. Step 1 represents the sampling enzyme cleavage step. The vertical dotted arrows indicate the positions of the sampling enzyme cleavage sites. Step 2 represents the pre-amplification step in which the sampled fragments are amplified. Step 3 represents the probing enzyme cleavage step. Step 4 represents the PCR primer design. The small horizontal arrows denote the PCR primers flanking the ESPs. Step 5 represents the PCR amplification step in which only the sample fragments that are not cleaved by the probing enzyme are amplified. The crossed circles represent the fragments that are not amplified.

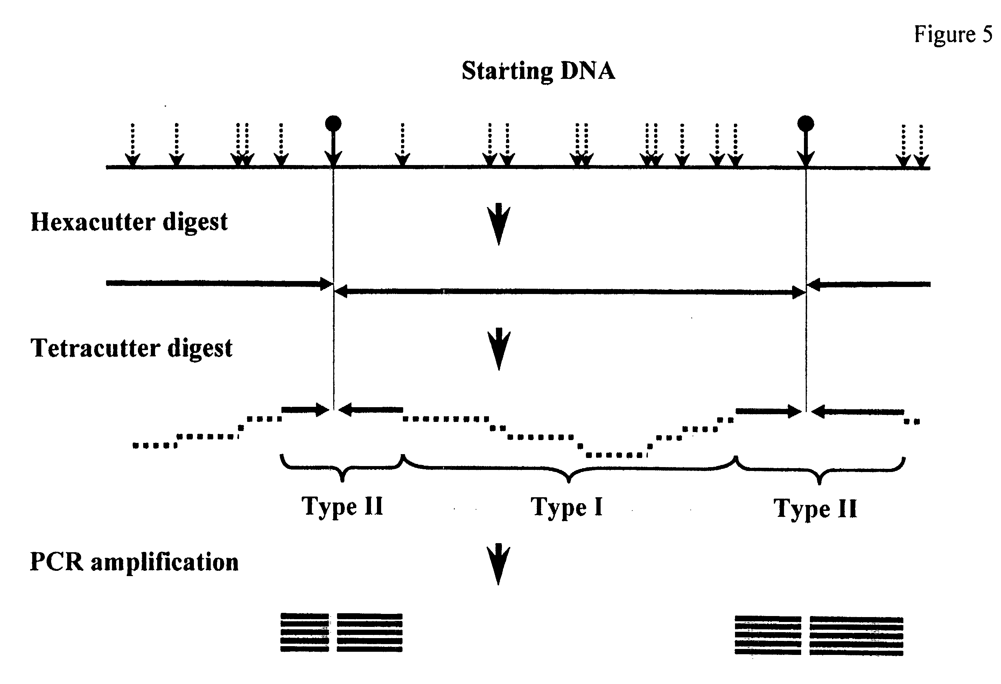

Figure 5: Graphic representation of target fragments produced by cleavage with a hexacutter (full arrows) and a tetracutter (dotted arrows) restriction enzyme. Two types of fragments are produced: type I fragments (dotted lines) carrying two tetracutter ends and type II fragments (full lines) carrying one hexacutter end (represented by the arrowhead) and one tetracutter end. Upon PCR amplification only the type I fragments are amplified.

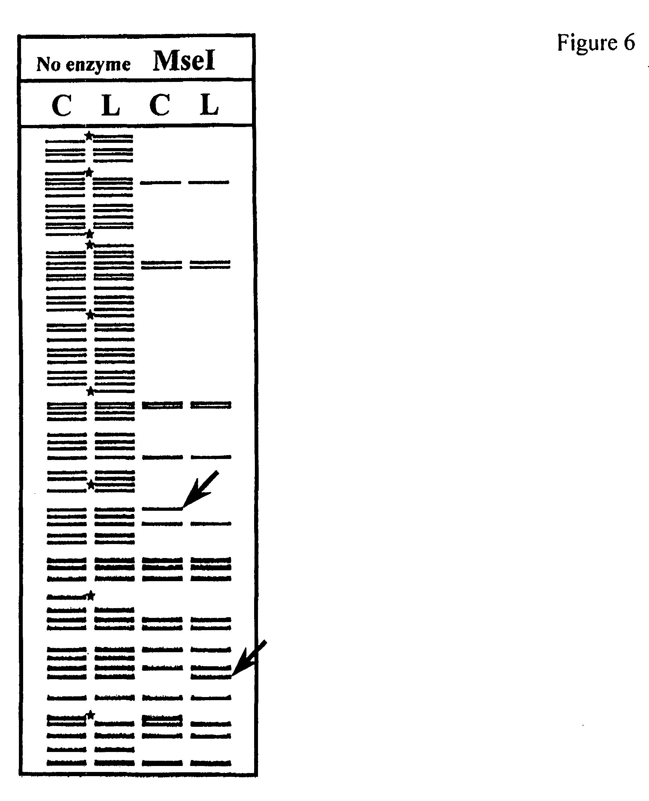

Figure 6: EcoRI-BfaI fragments from ecotypes Columbia (C) and Landsberg (L) obtained after selective amplification using EcoRI and Bfal AFLP primers with respectively 2 and 3 selective nucleotides. The fragment patterns were obtained respectively without probing enzyme (no enzyme) and after digestion with the Msel probing enzyme. It is noted that most of the larger fragments do not survive after Msel digestion, while the majority of the smaller fragments survive the treatment. The differences between the ecotypes Columbia (C) and Landsberg (L) observed after Msel digestion, marked by the arrows represent ESP carrying fragments. The differences found without Msel digestion, marked by the stars represent typical AFLP polymorphisms.

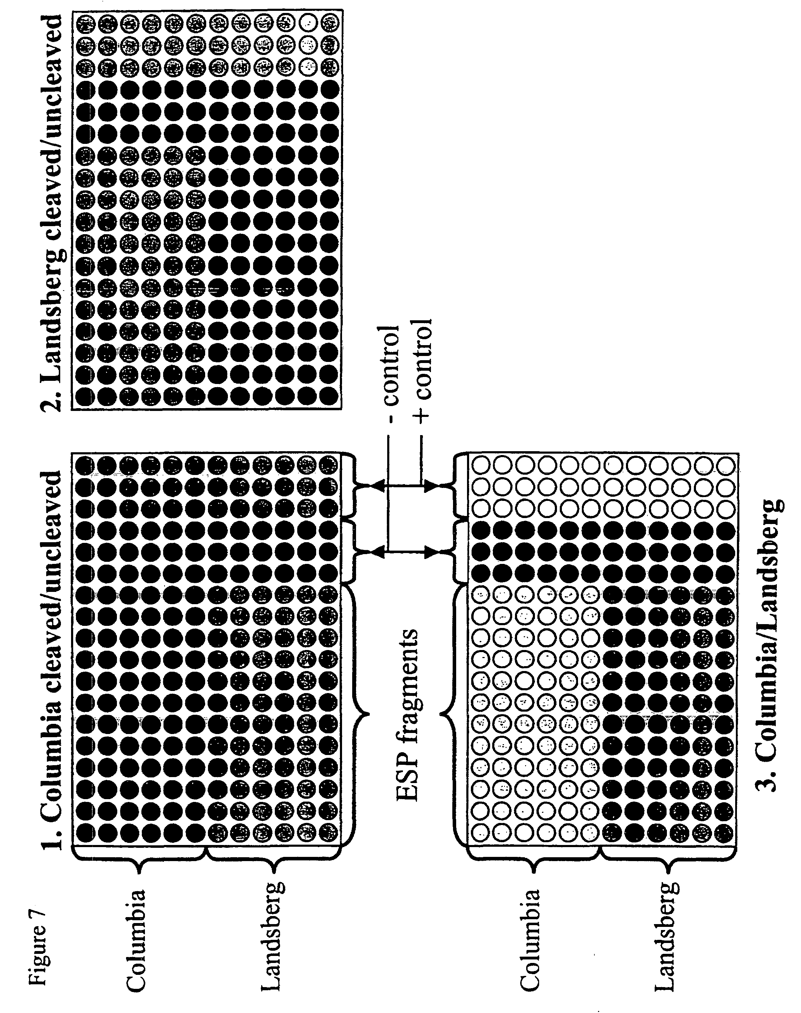

Figure 7: Hybridization patterns obtained on the Arabidopsis micro-arrays. The layout of the Arabidopsis micro-array is as follows: the left panel contains the ESP fragment probes derived from Columbia (upper half) and Landsberg (lower half), while the right panel contains the control monomorphic probes with respectively the negative control fragments (-control) always carrying a probing endonuclease site and the positive control fragments (+control) carrying no probing endonuclease site. The upper part of the figure shows the hybridization patterns obtained with uncleaved sample DNA, while the lower part of the figure shows the hybridization patterns obtained with cleaved sample DNA. The dark-grey circles code is as follows: light-grey circles represent hybridization with the Cy3-labeled fragments, dark-grey circles represent hybridization with the Cy5-labeled fragments, black circles represents hybridization with both the Cy3-labeled and the Cy5-labeled fragments, and open circles represent no hybridization. In this figure of a set of idealized results is presented. The hybridization patterns with the uncleaved sample DNA shows that all probes detect sequences in both ecotypes, while the hybridization patterns with the cleaved sample DNA show that the ESP fragment probes detect only the sequences in the respective ecotypes from which the ESP fragments were isolated. In addition, fragments carrying no site for the probing enzyme, detect sequences in both ecotypes, while fragments that always carry a site for the probing enzyme do not show a hybridization signal.

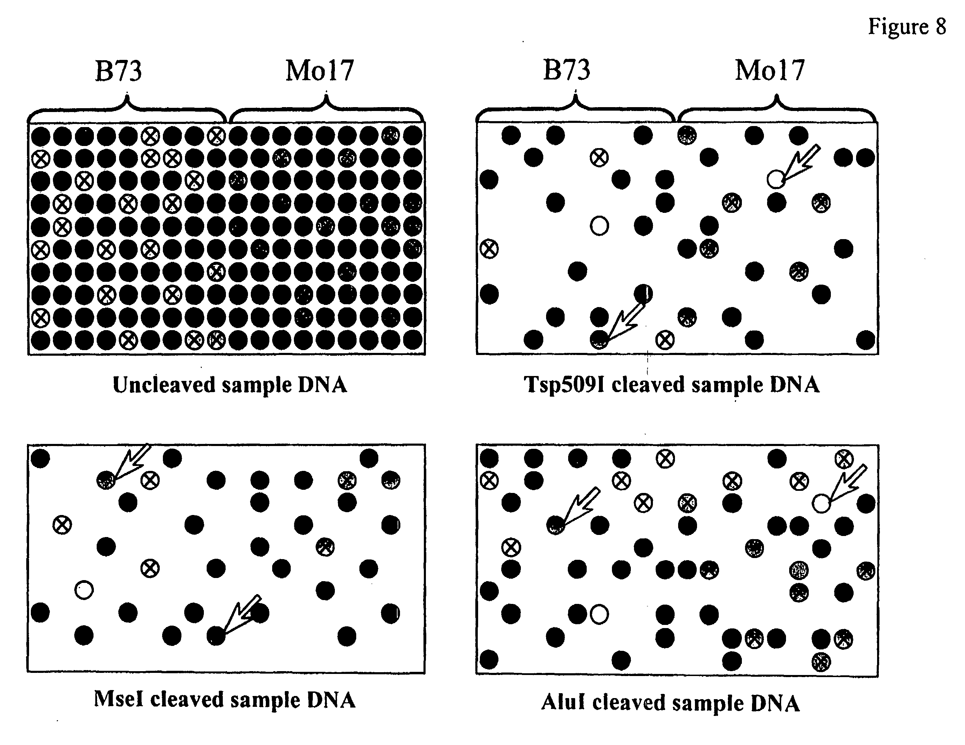

Figure 8: Hybridization patterns obtained on the corn micro-arrays. The layout of the corn micro-array is as follows: the left panel of probes contains random fragments derived from B73, while the right panel contains Mol7-fragments. The figure shows four hybridization patterns obtained with respectively uncleaved sample DNA, MseI-cleaved, Tsp509I-cleaved and Alul-cleaved cleaved sample DNA. The uncleaved sample DNA hybridization pattern shows probes that hybridize only to B73 (light-grey circles), respectively Mol7 (dark-grey circles) fragments, which represent polymorphisms resulting from mutations in the sample enzyme recognition sites. The cross in the circle indicates that these probes are eliminated from the analysis. The cleaved sample DNA hybridization patterns show that the majority of the probes do not give a hybridization signal, indicating that their cognate fragments are cleaved by the probing enzyme. Most of the probes giving a signal hybridize to both sample DNAs. Those that hybridize to only one of the sample DNAs and that were eliminated represent fragments carrying ESPs. The arrows denote the probes that were retained for further analysis.

### Detailed Description of the Invention

The term "SNP" means Single Nucleotide Polymorphism, i.e. a polymorphism involving the mutation of a single base-pair.

The term "ESP" means Endonuclease Site Polymorphism, i.e. a polymorphism involving two alleles one of which is cleaved by an endonuclease reagent while the other exhibits (at least partial) resistance to cleavage by the same endonuclease under the same conditions.

The phrase "(restriction) endonuclease reagent" refers to a reagent that consists of one or more enzymes and that cleaves nucleic acids with a certain specificity, i.e. cleavage involves recognition of a particular sequence or set of sequences in the target DNA. Endonuclease reagents include but are not limited to the common type II restriction enzymes.

The term "sampling endonuclease(s)" or "sampling enzyme(s)" refers to an endonuclease reagent used to derive sets of fragments from the sample DNA. The term "probing endonuclease(s)" or "probing enzyme(s)" refers to an endonuclease reagent used to probe the allelic state at specific ESP-sites.

The term "polymoiphism" refers to the existence of two or more alleles at significant frequencies (≥1%) in the population; polymorphism at a single chromosomal location constitutes a genetic marker.

The term "micro-satellite (DNA)" refers to a small array (often less than 0.1 kb) of tandem repeats of a very simple sequence, often 1 to 4 base-pair. Variability at such a locus is the basis of many genetic markers.

The term "mutation" means a heritable alteration in the DNA sequence.

The term "allele" refers to one of several alternative sequence variants at a specific locus.

The term "genotype" is commonly known to mean (i) the genetic constitution of an individual, or (ii) the types of allele found at a locus in an individual.

The term "haplotype" refers to the genotype at a series of linked loci on a single chromosome.

The term "sample DNA" or "sample fragments" refers to the set of fragments or amplicons derived from the starting DNA by the RAA method.

The term "zygosity" refers to the homozygous or heterozygous state.

The term "homozygosity/homozygous" refers to the presence of identical alleles at a locus.

The term "heterozygosity/heterozygous" refers to the presence of different alleles at a locus.

The term "CpG" means a dinucleotide with a cytosine at the 5'-side and a guanine at the 3'-side. CpG is relatively rare in mammalian DNA because of the tendency for the cytosine to be methylated and subsequently mutate to thymine by deamination.

The term "ecotype" refers to a naturally occurring (plant) variety; race.

The term "bi-allelic" refers to a polymorphic locus characterized by two different alleles.

The terms "microarray" and "(DNA-)chip" refer to a multitude of spatially addressable nucleic acids that serve as probes. The microarray may be used in the form of a planar solid support, a bead, a sphere, or a polyhedron. Fabrication is done either by in situ combinatorial synthesis of oligonucleotides using photolithography, or by robotic spotting of off-chip prepared DNA onto a solid surface.

The methods of the present invention differs conceptually from previously described restriction enzyme-dependent assays *(supra)* that essentially detect a fragment length polymorphism. With the present method, starting DNA is restricted prior to the amplification reaction and, rather than analyzing the obtained amplification product, the presence or absence of amplification is measured to determine the allelic state at an ESP site. The treated DNA is preferably amplified by using a polymerase chain reaction and is preferably analyzed by means of hybridization against arrays of probe DNAs. With the present method, a sample-amplicon, and consequently a hybridization signal, is either present or virtually absent. This feature represents a major advantage in that it results in a more accurate distinction between variable nucleotides than is possible by differential hybridization to allele-specific oligonucleotides, and because it greatly facilitates the identification of a set of generally useful hybridization conditions. Also, the methods of the invention permit the use of both oligonucleotides as well as DNA fragments as probe DNAs. While hybridization to arrays allows the simultaneous analysis of a large number of ESPs, it should be clear that the amplification of sample DNA, treated with probe restriction endonuclease reagent, can be analyzed by any of a variety of methods well known in the art. In these methods, an ESP is identified either by the presence of a recognition site for the probe restriction endonuclease reagent (which will result in the failure of the sample DNA to amplify) or by the loss of a recognition site which will allow amplification of an otherwise unamplifiable sample DNA. Alternative methods include, but are not limited to, gel-electrophoretic analysis, and the TaqMan assay [Holland P. M. *et al. Proc.* *Natl. Acad. Sci.* **88**: 7276-7280 (1991); with the latter assay detection is done during rather than after the amplification reaction].

One of the advantages of the method of the invention is the ability to calibrate the measured signal against that obtained in a control experiment where digestion with the probe restriction endonuclease reagent is omitted. Comparison of the respective hybridization signals, following various corrections and normalization procedures, is essential for the genotyping of ESPs and the accurate determination of the zygosity. The cleaved and uncleaved material can, in principle, be hybridized separately but a preferred method consists of hybridizing a mixture of the differentially labeled samples to the same array. The present invention is exemplified by several specific formats described below.

**(I) Format-I RAA:** Choice of sampling and probing restriction endonuclease reagents. In one of its embodiments the present invention is directed to methods for detecting ESPs in a "restricted amplicon assay" (RAA) which comprises preparing concomitantly amplifiable restriction fragments from the starting DNA (sample DNA). When generating discrete sets of DNA fragments from genomic DNA, the following parameters are important: the average fragment size and the total number of fragments. The optimal fragment size for use in the methods (and materials) of the present invention is a trade off; the fragments must be sufficiently small for amplification with roughly equal efficiency (in general ≤500 base pairs) and large enough for having on average one cleavage site for the probing endonuclease reagent. In addition to average fragment size, the number of fragments determine the complexity of the sample DNA which is critical in view of the limitations of the detection sensitivity of micro-array hybridization. In general, the current state of the art of microarray hybridization is such that the number of sample fragments should not exceed 100,000. All of the above-mentioned requisites can be met by the appropriate choice of sampling and probing enzymes. A preferred method of the present invention to prepare sample DNAs (amplicons) involves the use of two different sampling enzymes, a rare cutter endonuclease (*e.g.*, hexacutter) combined with a frequent cutter endonuclease (*e.g.*, tetracutter), as described in EP 0 534 858 A1 which describes a method called AFLP. As can be seen from Figure 5, the rare cutter enzyme produces large fragments that upon cleavage with the frequent cutter enzyme are cut into a number of smaller fragments. This dual cleavage generates two types of fragments: the majority having both ends produced by the frequent cutter (type I) and a minority of fragments having a rare cutter end and a frequent cutter end (type II). After ligating different adapters to each of the ends and using appropriate primers targeted to the ends of the fragments, only the type II fragments will be amplified efficiently (see Figure 5). The type I fragments amplify with greatly reduced efficiency presumably because the synthetic sequences at the two ends constitute an inverted repeat. In general the type II fragments will amplify synchronously using a single PCR primer pair that attaches to the ends of the fragments. The size limit is typically around 500 base pairs, but can be increased by using a different DNA polymerase and other reaction conditions. Thus, as outlined above the number of amplifiable fragments will be determined primarily by the choice of the rare cutter restriction enzyme. By approximation, this number equals two times the number of cleavage sites for the rare cutter. In a preferred embodiment, restriction enzymes recognizing 6 nucleotides (hexacutters) or more are used as rare cutters. The use of a frequent cutter recognizing 4 nucleotides (tetracutter) as second sampling enzyme results in the production of fragments in the optimal size range for co-amplification. As probe restriction endonuclease reagents, different tetracutter or pentacutter enzymes can be used. The probe restriction endonuclease reagent and the frequent cutter sampling enzyme should preferably be chosen such that the ratio of the cleavage frequencies of probing over sampling reagent is >0.5 and < 3. This will ensure that a substantial fraction of the target fragments are cleaved once by the probing enzyme. It is noted that ESPs cannot be genotyped when the fragments are cleaved more than once by the probing enzyme. Also, it should be recognized that cleavage with the probe restriction endonuclease reagent results in a significant reduction (typically 2-4 fold) of the fragment complexity.

Alternative schemes - different from the one described above - that meet the requisites of sample complexity, average fragment size, and occurrence frequency of the probe reagent and that will perform equally well, will be readily apparent to one of ordinary skill in the art. Alternative schemes may include the use of pairs of frequent cutters, followed by selective amplification (described in EP 0 534 858 A1), or the use of type IIs restriction enzymes. Type US restriction enzymes are characterized by an asymmetric recognition sequence. Most of these enzymes cleave at a defined distance to one side of the recognition site and generate single stranded overhangs that have different sequences. Ligation of adaptor sequences that are complementary to only one type of overhang allows the amplification of specific subsets of fragments [Kikuya Kato, *Nucleic Acids Res.* **23:** 3685-3690 (1995)]. With this strategy the set of fragments obtained with the sampling enzymes can be broken up in a defined number of complementary and roughly equally complex subsets. Thus, with these enzymes it is possible to tune the complexity of the sample. The same strategy can be applied by making use of type II enzymes that have an interrupted palindromic recognition sequence.

**Type of mutations detected by format-I RAA:** In essence the method of the invention aims to detect mutations affecting the recognition sequences of the site-specific probe endonuclease reagents. When the probe enzyme cleaves a sample fragment, it is prevented from being amplified and as a consequence the fragment will not give a hybridization signal with its cognate probe. Mutations affecting the recognition sequence of the probe enzyme will allow amplification of the sample fragment and will restore the hybridization signal. It is recognized that mutations other than those affecting the probe enzyme recognition sites may affect the hybridization than those affecting the probe enzyme recognition sites may affect the hybridization signals. In particular, mutations affecting the recognition sites of the sampling enzymes may also lead to a loss of hybridization signal. Consequently, the mere detection of a hybridization difference between two samples does not qualify the difference as being due to an ESP for the probing enzyme. For this one must also assay the two samples without probing enzyme cleavage; only those differences that are correlated with the cleavage by the probing enzyme qualify as genuine ESPs as defined according to the present invention. Therefore, a preferred embodiment of the methods of the present invention comprise the comparison of the hybridization signals obtained with and without cleavage of the same starting material by the probe endonuclease reagent. Preferably, the digested and undigested sample DNAs are differentially labeled such that equivalent amounts of the material can be mixed and hybridized against the same array of probes. It is noted that a further advantage of measuring the relative hybridization signals obtained with digested and undigested sample DNAs, is that the signal given by the undigested sample DNA serves as an internal control for correcting variations in amplification and hybridization.

**Identification and design of informative probes to detect ESP-harboring fragments.** In a preferred embodiment of the present invention sample DNAs (amplicons) are hybridized to micro-arrays comprising a set of probe DNAs which are designed such that each probe will hybridize specifically to one sample DNA fragment. For each set of sample DNA fragments a specific set of probes are developed that will detect all the ESPs present in the set of sample DNAs. Since in most applications only a (minor) fraction of the sample DNAs will actually carry an ESP for a particular probing reagent, the set of probe DNAs will preferably consist of a subset of the sample DNA fragments that are informative in that they hybridize to ESP-harboring sample fragments. Preferably, the probes are highly specific for the ESP-carrying sample fragments, and do not cross-hybridize with other fragments in the sample. This feature is verified by testing the candidate probes in control hybridization assays. When developing or designing the probes care should be taken to avoid hybridization of the labeled primer used to amplify the sample fragments. When the probes correspond to a subset of the sample fragments, preferably an alternative set of adaptors should be used for their amplification.

The sections below describe different approaches that may be used to assemble sets of unique probe DNAs for fabricating the micro-arrays. Three alternative approaches are presented, and their choice is determined primarily by the degree of nucleotide sequence variation, and hence the ESP frequency, present in the species under study.
(1) Direct screening. When the ESP frequency is high, such that 10 % or more of the sample fragments carry ESPs, a realistic approach for assembling ESP probes is to array individual sample fragments and test which of them detect an ESP in the test material under study. The advantage of this approach is that the same set of fragments can be tested with different probe enzymes. After the screening one will retain only those probes that yield a clear-cut difference in hybridization between the different test DNAs. This approach is illustrated in Example 2.
(2) Gel-based screening. With genomic DNA exhibiting intermediate ESP frequencies (a few %), useful probes can be identified with a gel-based screening approach in which the ESPs are identified by comparing the patterns of sample fragments obtained from cleaved and uncleaved genomic DNA of various individuals. The polymorphic fragments can then be isolated from the gel and cloned or amplified. In a second phase, these probe-fragments are verified in a micro-array hybridization assay. This approach is illustrated in Example 1.
(3) Batch-wise hybridization selection method. Since both approaches described above are inefficient and labor intensive when the ESP frequency is low (<1%), it is advantageous to directly select or enrich ESP-carrying fragments. Such an approach is described in greater detail in Example 3.

The methods of the invention can be used with any type of micro-array: spotted ESP-carrying fragments, spotted oligonucleotides or oligonucleotides synthesized on solid supports using photolithography [Fodor S. P. A. *et al., Science* **251**: 767-773 (1991)]. Oligonucleotide probes can easily be designed based on the nucleotide sequences of the ESP-carrying fragments. Also, the methods of the invention are not limited to the use of planar arrays containing spatially addressable probes. A person of skill in the artwill recognize that the methods may alos employ a multitude of identifiable solid phase particles (e.g. beads, spheres, and polyhedron), each carrying a different probe. Examples of such use are described by Fulton, R. [U.S. Patent No. 5,736,330] and Mandecki, W. [ U.S. Patent No. 5,736,332].

### (III) Format-II RAA General outline

The 'format-I RAA' - as described above - can be converted to a 'format-II assay' when sufficient sequence information of ESP-containing sample fragments becomes known. Format-II RAAs can also be designed on the basis of the known sequences of genomic regions that harbor an ESP and that are available through publicly accessible databases. The approach involves the targeted sampling of starting material and consists of the design of dedicated primer pairs that flank the ESP sites. Like in format-I RAA, if the site is intact, the starting DNA will be cleaved and no PCR product will be generated. Only when the site is mutated will the amplicon be generated. In practice, multiple ESP-containing genomic regions are co-amplified after cleavage with the probing restriction endonuclease reagent. The ultimate sample DNA used in the hybridization reaction is composed of several such multiplex PCR reactions pooled together. The feasibility of this approach is evidenced by the recent paper of Wang *et al., Science* **280**: 1077-1082. The methods for format-II RAA described here are identical to the approach described by *Wang et al.,* in the way certain allelic regions are co-amplified, but fundamentally different in the way they are diagnosed. The present method takes advantage of the clear distinction between having or not having an amplicon depending upon the allelic state of the endonuclease target site. The Wang *et al.* approach in contrast relies on the detection of a hybridization difference as a result of a single nucleotide variation in the PCR product. This requires a much more elaborate and redundant hybridization assay.

Similar to format-I RAA, a preferred method consists of comparing the hybridization signals obtained with and without cleavage with the probe restriction endonuclease reagent. Preferably, the respective amplification reactions are differentially labeled such that the resulting amplicons can be mixed and hybridized against the same array of probes.

Preferred methods of the format-II RAA are those wherein - of each PCR primer pair - that primer that remained unlabeled is used as hybridization probe for the corresponding amplicon. This ensures that the excess unincorporated labeled primer as well as the primer extension products obtained with this primer cannot anneal to the arrayed probe. Also, the unlabeled PCR primer is complementary to the labeled strand of the amplicon.

Furthermore, the format-II RAA method provides a means to monitor mutations in specific genes or loci in addition to scanning the entire genome. Indeed, sets of PCR primers that target ESPs in a specific gene or chromosome region can be assembled.

**An RAA assay with positive detection of both alleles:.** It is recognized that the 'present/absent-score' of the RAA assay cannot (always) distinguish between different mutations that can affect cleavage by the probe restriction endonuclease reagent. In practice, an ESP should not be assayed when available evidence indicates the existence of two or more such mutations at significant frequencies in the population.

In a preferred embodiment the present invention is directed to the on detection of SNPs that result in the simultaneous loss and gain of a restriction enzyme recognition site, *i.e.* both alleles are associated with a different recognition site. HgaI (GACGC) and SfaNI (GATGC) are an example of such reciprocal sites. Use of both probing endonuclease reagents in side-by-side experiments excludes alternative alleles and results in easy determination of the zygosity (refer to Example 4).

**Multi-allelic haplotyping:** A single ESP represents a bi-allelic marker, which is less informative than a variable micro-satellite, which has multiple alleles. It is possible however to compensate for the lower information content by identifying several ESPs on a specific chromosomal region. Format-II RAA lends itself readily to such an approach and involves the design of a primer pair that encompasses a region with a single site for the various selected probe endonuclease reagents. It should be recognized as one of the advantages of the present method that multiple ESPs on a sample amplicon can be interrogated with a single probe. Furthermore, use of the probing enzymes, either separately or in various combinations, in parallel experiments allows the construction of the haplotypes for the ESPs under study. In general, the statistical associations between traits and specific chromosome regions may be more apparent when haplotypes rather than individual markers are used.

### (III) Format-III RAA:

In a general sense, the format III RAA represents a method of choice for very high-density SNP genotyping because it provides a means to overcome the intrinsic limitations of both the format-I RAA and the format-II RAA. This is essentially achieved by performing a stepwise amplification involving a pre-amplification of sample fragments followed by amplification using multiplexed specific primers. The principal advantage of the pre-amplification step is to reduce the complexity of the starting DNA, and thus to provide a more favorable starting point for performing multiplex PCR reactions. It is noted that this improvement is generally applicable to any multiplex PCR reaction, and is not limited to the methods of the present invention. Such an approach can also be used when for example SNPs are genotyped using the methods described by Wang *et al.*

The principal limitation of the format-I RAA lies in the complexity of the sample DNA that is hybridized to the microarray. Because the second round of amplification in format-III yields only very small amplicons, which are all informative, there is no longer a limitation in number of sample fragments that are interrogated. In fact the entire genome may be sampled in a series of parallel pre-amplification reactions and the amplicons generated in the different multiplex PCR reaction can then be pooled together and hybridized to the microarray.

Likewise, the format-III RAA represents preferred methods of format-II RAA, especially when the ESPs under study are located on fragments generated by one set of sampling endonuclease reagents. Such stepwise amplification comprises the co-amplification of sample fragments with a single pair of primers, followed by the selective amplification of sets of specific ESP-containing regions (see Figure 5). The principal advantage of the format-III RAA over format-II RAA is that the initial amplification of the sampling fragments - representing only a fraction of the total genome - lowers the amount of starting material required to interrogate a very large numbers of ESPs. Also, the approach will facilitate the multiplex amplification of the ESP-specific amplicons and, consequently, yield a more robust assay.

One preferred embodiment of the format-III RAA is its use to genotype large numbers of ESPs identified through the use of the format-I RAA. Indeed, format-I RAA offers a rapid means to discover large numbers of ESPs in any biological species where no large body of sequence information is or will be available. Format-I RAA enables one to discover many sets of ESPs for a number of different probing enzymes. Using the format-I RAA, each set of ESPs must be assayed on a different microarray, because otherwise signals for the same sample fragment will overlap with one another, and thus preclude the proper ESP genor type to be determined. Using the one another, and thus preclude the proper different probing be determined. Using the format-III RAA, the ESPs identified with different probing enzymes are now assayed together on one single microarray, without overlap between the different ESPs. The reason is that the overlap in the format-I RAA is caused by the non-informative sample fragments that are always co-amplified with the ESP fragments. These are eliminated from the mixture by the specific PCR amplification. This embodiment is illustrated in Examples 2 and 3.

Another preferred embodiment of the format-III RAA is its use to genotype large numbers of SNPs identified in high-throughput sequencing of genomic DNA from different individuals from a given species. Given the generally recognized importance of SNPs for the development of high-resolution genotyping methods, sequenced SNPs can be expected to accumulate in large numbers in publicly available databases in the near future. In particular, in the field of human genetic analysis, SNPs will be discovered at a rapidly increasing rate through the massive genome sequencing programs now in progress. A similar evolution may be anticipated for many other species. Hence we decided to perform an *in silico* analysis of known human SNPs to further investigate the potential of the invention. More particularly we have analyzed the 3,358 SNP sequences present in the SNP database of the Whitehead Institute [Wang *et al., Science* **280**: 1077-1082 (1998)]. We have determined how many of these SNPs represent an ESP for each of 34 known palindromic and non-palindromic tetra- and penta-nucleotide restriction recognition sequences. When extrapolating this number to the total number of ESPs in the human genome - assuming a grand total of 3 million ESPs - it appears that the number of detectable ESPs per probing restriction enzyme is in the range of 25.000 to 150.000. A cumulative analysis reveals that 53% of the SNPs affect at least one of the 34 restriction sites; a total of 28 % affect the recognition site for one of the available tetracutter enzymes. The principal conclusion from this analysis is that many of the considered enzymes - used as probing enzymes according to the methods of the present invention - will interrogate sufficient SNPs to be able to built a high-density map of the human genome. It should also be noted that the use of multiple probing enzymes is easily accommodated in the targeted assay because the sample has to be subdivided anyway over a number of parallel multiplex PCR reactions. This embodiment is illustrated in Example 4.

It is noted that the format-III RAA may be performed according to different procedures. One such procedure is diagrammed in Figure 5, in which the test DNA is first sampled using a sampling endonuclease reagent, pre-amplified and then treated with the probing endonuclease reagent. Variations on this procedure are readily recognized by those skilled in the art and include for example, concomitant treatment of the test DNA with both the sampling and the probing endonuclease reagents and the preparation of sampled DNA fragments using arbitrary PCR priming methods [Williams *et al., Nucleic Acids Res.* **18**: 6531-6535 (1990)]. Note that in case the treatment with the probing endonuclease reagent is performed prior to the pre-amplification, the subsequent amplification can be performed with any pair of PCR primers directed against the ESP carrying fragments, and thus overcoming the limitation of using PCR primers flanking the ESPs.

**Table I.**

| Analysis of 3,358 SNPs in the Whitehead SNP database. The table lists the number of SNPs that represent an ESP for various probing enzymes. The last column shows the estimated number of ESPs for each enzyme in the entire human genome (refer to text for details}. | | | | |
|---|---|---|---|---|
| Type of probing reagent | enzyme | site | ESPs | total number of ESPs |
| Tetracutter enzymes | Tsp509I | AATT | 122 | 109.000 |
| | MaeII | ACGT | 106 | 95.000 |
| | AluI | AGCT | 98 | 88.000 |
| | NlaIII | CATG | 158 | 141.000 |
| | MspI | CCGG | 77 | 69.000 |
| | BstUI | CGCG | 27 | 24.000 |
| | BfaI | CTAG | 67 | 60.000 |
| | Sau3A | GATC | 58 | 52.000 |
| | HinPI | GCGC | 49 | 44.000 |
| | HaeIII | GGCC | 52 | 46.000 |
| | Csp6I | GTAC | 71 | 63.000 |
| | TaqI | TCGA | 50 | 45.000 |
| | MseI | TTAA | 109 | 97.000 |
| Pentacutter enzymes | Tsp4CI | AGNCT | 114 | 102.000 |
| | BssKI | CCNGG | 79 | 71.000 |
| | DdeI | CTNAG | 122 | 109.000 |
| | HinfI | GANTC | 77 | 69.000 |
| | Fnu4HI | GCNGC | 71 | 63.000 |
| | Sau96I | GGNCC | 64 | 57.000 |
| | MaeIII | GTNAC | 70 | 63.000 |
| Non-palindromic enzymes | AciI | CCGC | 111 | 99.000 |
| | MnII | CCTC | 175 | 156.000 |
| | BbvI | GCAGC | 65 | 58.000 |
| | BsmAI | GTCTC | 67 | 60.000 |
| | BsmFI | GGGAC | 39 | 35.000 |
| | FokI | GGATG | 66 | 59.000 |
| | HgaI | GACGC | 31 | 28.000 |
| | PleI | GAGTC | 39 | 35.000 |
| | SfaNI | GCATC | 51 1 | 46.000 |
| | AlwI | GGATC | 37 | 33.000 |
| | BsrI | ACTGG | 76 | 68.000 |
| | HphI | GGTGA | 69 | 62.000 |
| | MboII | GAAGA | 85 | 76.000 |
| | TspRI | CAGTG | 94 | 84.000 |

The following illustrative examples were chosen to represent the spectrum of genomic complexities and the spectrum of degrees of genetic variation which are susceptible to analysis using the methods of the present invention:
Example 1 describes analysis of Arabidopsis (low genomic complexity, low genetic variation).
Example 2 describes genetic analysis of corn (high genomic complexity, high genetic variation).
Examples 3 and 4 describe genetic analysis in humans (high genomic complexity, low genetic variation).

Numbers given in the examples, and that relate to the occurrence frequency of certain restriction sites as well as the average size of the generated fragments are in part based on computer simulations using publicly available DNA sequences.

### Example 1

### Genetic Analysis in Arabidopsis

In this example, a fragment analysis-based approach is used to generate a set of genomic fragments carrying ESPs between the Arabidopsis ecotypes Landsberg and Columbia, which are commonly used for genetic studies in the model organism. Arabidopsis is an example of a low complexity genome (size ^{~}120 Mb), and the two ecotypes exhibit a moderate level of genetic variability. Previous studies have revealed that the average nucleotide sequence variation between the two ecotypes is in the order 1 polymorphism in 150 nucleotides. Consequently, the fraction of fragments expected to carry an ESP for tetranucleotide recognizing restriction enzymes is expected to be in the range of 2.5 % (1:40). With such a low frequency, it is helpful to use a selection procedure to isolate the rare fragments containing ESPs.

In essence the procedure described in this example comprises the following steps:
4) Identification of a set of about 200 genomic fragments carrying Landsberg/Columbia ESPs using a gel-electrophoretic approach.
5) Isolation and characterization of the ESP carrying DNA fragments (ESP fragments).
6) Generation of micro-arrays with the ESP fragments
7) Confirmation of the ESPs by hybridization.

### Step 1. Identification of ESP fragments,

*Sampling enzymes.* In the present example EcoRI, a restriction enzyme recognizing 6 nucleotides (hexacutter), in combination with Bfal, a restriction enzyme recognizing 4 nucleotides (tetracutter), are chosen as sampling enzymes. From the random frequency of occurrence of 6 nucleotide sequences (every 4,000 bases), the number of sites for hexacutter restriction enzymes in this genome is predicted to be in the range of 30,000. In addition to cleavage with a hexacutter, the genomic DNA is also cut with a tetracutter so as to generate PCR amplifiable fragments of an average size of a few hundred base pairs. Cleavage with the two enzymes gives rise to two types of fragments: a majority of fragments resulting from cleavage by the tetracutter enzyme alone and a smaller set of fragments produced by the two enzymes (see Figure 5). Since the majority of the hexacutter fragments will give rise to two fragments having a hexacutter end and a tetracutter end (*see* Figure 5), this procedure will yield a mixture of about 60,000 fragments of this type. Upon amplification using the procedure described below only the fragments carrying a tetracutter end and a hexacutter end are amplified efficiently (Figure 5).

*Probing enzymes.* As probing enzymes many different tetracutter . enzymes can be used. Ideally, the probing enzyme cleaves most of the sample fragments once. Because plant DNA has a high AT content, the preferred tetracutters are those that have an AT bias in their recognition sequence. In general, the choice of an optimal tetracutter may be determined by particular features of the genome being analyzed (*e.g.,* AT and GC content). In the present example, MseI (recognition site = TTAA) was chosen. Tsp509I (recognition site = AATT) is an alternative. It is also conceivable to use mixtures of two or more tetracutter enzymes. The EcoRI BfaI sample/target fragments that are cleaved and not cleaved with the MseI probing enzyme are referred to as cleaved and uncleaved sample/target DNA, respectively.

*Screening for ESP carrying fragments.* To detect ESP fragments, subsets of uncleaved and cleaved EcoRI-BfaI sample fragments from both ecotypes are amplified and the amplicons are compared following gel-electrophoretic fractionation. Subsets of the EcoRI-BfaI sample fragments are selectively amplified as described [Vos, P. *et al., Nucleic Acids Res.* **23**: 4407-4414 (1995); Zabeau, M. and Vos, P., European Patent Application EP 0534858 (1993). Given the complexity of the sample (⁻50*,*000 fragments), the selective amplifications are performed with EcoRI and BfaI primers having two and three selective nucleotides, respectively. This equals 1024 (16 x 64) different selective amplification reactions.

The experimental procedure described by Vos P. *et al.* is followed except that the template fragments are incubated at 65°C during 10 minutes to heat-inactivate the T4 ligase enzyme, and, when applicable, digested with the probing enzyme prior to amplification. The structures of the EcoRI and Bfal adaptors are as follows [*see, e.g.,* Vos, P. *et al., supra*):

The EcoRI (radiolabeled by 5'-phosphorylation) and BfaI primers, having two and three selective nucleotides, respectively, have the following sequences (where N represents A, C, G, or T):

Using these reagents, most of the obtainable target fragments contain a cleavage site for the probing enzyme and, consequently, will not be amplified when the target DNA is cleaved. Most of the fragments that survive the treatment with the probing enzyme occur in both ecotypes, and thus carry no ESP. Occasionally fragments are found that appear in both ecotypes when the target DNA is not digested and that are present in only one of the two ecotypes after digestion. These represent true ESPs for the probing enzyme. In addition, fragments will also be found that show typical AFLP-polymorphism between the two ecotypes [Vos, P. *et al. Nucleic Acids Res.* **23:** 4407-4414 (1995)]. Such polymorphisms are apparent in the fragment patterns obtainable with the undigested sample DNAs. A typical result is shown in Figure 6 in which the electrophoretic patterns are shown of selectively amplified EcoRI-BfaI fragments from the Ecotypes Columbia and Landsberg obtained without and with digestion with the MseI probing enzyme.

Systematic comparison of the patterns of ecotypes Columbia and Landsberg before and after digestion, allows the identification of EcoRI-BfaI sample amplicons that carry an ESP for the probing enzyme. Using MseI as probing enzyme, it is estimated that a total of ^{~}200 polymorphic fragments which are present in only one of the ecotypes can be identified.

### Step 2. Isolation and characterization of ESP fragments.

Each of the ESP polymorphic fragments is eluted from the gel-matrix, re-amplified and cloned into a suitable plasmid vector (e.g. TA cloning system; Invitrogen, Carlsbad, CA, U.S.A.). In each case, two clones are selected for sequence determination. Most duplicate clones will yield the same sequence. Duplicate clones that gave different sequences were not retained for further work. Since the nucleotide sequence of over one third of the Arabidopsis genome is available in the public databases (*e.g.*, Genbank), the chromosomal location of one third of the ESP fragments can be determined by matching the fragment sequences to the genomic sequence. Furthermore since the genomic sequence is derived from ecotype Columbia, we expect a perfect match with the fragment sequences isolated from the same ecotype. The sequences of the fragments isolated from ecotype Landsberg will reveal single nucleotide differences, amongst which the potential restriction site mutations, affecting the MseI recognition sites, should be apparent.

In addition to the ESP polymorphic fragments, a number of non-polymorphic control fragments are processed in the same way. Two types of such control monomorphic fragments are isolated: fragments that do not carry a site for the probing enzyme and fragments that carry a site for the probing enzyme in both ecotypes. These fragments will serve the purpose of verifying the hybridization on the micro-arrays.

### Step 3. Fabrication of ESP micro-arrays.

*Micro-arrays of amplified fragments.* The insert DNAs from the sequence verified clones are amplified, e.g. with the use of non-selective EcoRI and BfaI primers. PCR products are verified by agarose gel electrophoresis and retained if a single product of the correct mobility was present. Following ethanol precipitation, the resuspended PCR products are arrayed at high density on standard glass slides (25 x 76 mm) using either the Multigrid robotic spotter (GeneMachines^{TM}, Genomic Instrumentation Services Inc., Menlo Park, CA, U.S.A.) or the BioChip Arrayer^{TM} (Packard Instrument Company, Meriden, CT, U.S.A.). The DNAs are spotted in a logical order with respect to the ecotype from which the fragments were isolated (upper and lower panel) as shown in Figure 7. In addition, a set of DNAs from monomorphic control fragments was spotted next to the ESP fragment DNAs (right panel in Figure 7).

*Micro-arrays of oligonucleotides.* Based on the nucleotide sequences of the ESP fragments, oligonucleotides can be designed that can serve as hybridization probes to specifically detect each amplified sample fragment. The oligonucleotide probe should preferably match with a sequence that is located to one side of the ESP, opposite the side where the sequence targeted by the labeled primer is located. In this way the background is minimized because the linear amplification products generated by the labeled primer following digestion with the probing enzyme are not detected. The ESP fragment specific oligonucleotides are spotted in a micro-array format in exactly the same way as the amplified ESP fragments.

### Step 4. Micro-array-based detection of ESPs.

*Prepararion of the sample DNAs.* For each ecotype, sample DNA is prepared in two different ways. Genomic DNA, digested with the sampling restriction enzymes EcoRI and BfaI, was amplified either as such or after cleavage with the probing enzyme MseI. The amplification reactions are performed with a fluorescently labeled EcoRI primer and an unlabeled BfaI primer, both without selective nucleotides. The EcoRI primer is labeled by incorporation of Cy3(green)- and Cy5(red)-amidites during primer synthesis (Amersham Pharmacia Biotech, Uppsala, Sweden). For both Columbia and Landsberg, the cleaved sample was amplified with a Cy3-primer while the uncleaved fragments were amplified with a Cy5-Iabeled EcoRI primer. In addition, the Landsberg digested material was also amplified with a Cy5-labeled EcoRI PCR primer. Three different hybridization solutions are then prepared by mixing equal amounts (i.e. equal volumes) of the Cy3- and Cy5-labeled amplification reactions: one from the Columbia cleaved and uncleaved samples, a second from the Landsberg cleaved and uncleaved samples, and a third by mixing the differentially labeled cleaved samples of both ecotypes.

In case arrays of PCR products, rather than oligonucleotides, are used as probes (refer to step 3), the co-amplification of the EcoRI-BfaI sample fragments is preferably accomplished with a pair of adaptors that differs from those attached to the arrayed probes, The alternative EcoRI and BfaI adaptors have the following structure:

The cognate non-selective EcoRI and BfaI primers have the following sequences:

***Micro-array hybridization.*** Each of the hybridization solutions is allowed to hybridize to the arrayed probes using protocols well known in the art. The experimental conditions depend primarily on the nature of the probes, PCR-amplified fragments versus oligonucleotides. Both types of experiments are amply described in literature: Wodicka, L. *et al., Nature Biotechnol.* **15**: 1359-1367 (1997); Lockhart, D. J. *et al., Nature Biotechnol.* 14: 1675-1680 (1996); DeRisi, J. L. *et al., Science* **278**: 680-686 (1997); Shalon, D. *et al., Genome Res.* **6**: 639-645 (1996); Piétu, G. *et al., Genome Res.* **6**: 492-503 (1196); Chee, M. *et al., Science* **274**: 610-614 (1996); Wang D.G. *et al.,* ***Science* 280**: 1077-1082 (1998); **Winzeler** E. A. *et* ***al.,*** *Science* **281**: 1194-1197 (1998).

A laser scanning system (ScanArray 3000; General Scanning Inc., Watertown, MA, U.S.A.) is used to detect the two-color fluorescence hybridization signals from the micro-arrays at a resolution of 10 micron per pixel. A separate scan is carried out for each of the two fluorophores used. Scanning parameters and laser power settings are adjusted to normalize the signal in the two channels (channel-1/Cy3; channel-2/Cy5). The obtained digital images were analyzed using the ImaGene^{TM} image analysis software (BioDiscovery Inc., Los Angeles, CA, U.S.A.). The extracted quantitative data are transferred to a spreadsheet for further analysis.

The present hybridization experiment is essentially set up as a confirmation of the gel-electrophoretic data (refer to step 1), and has, therefore, a predictable outcome. In addition, a number of control probes are included on the biochip that detect monomorphic EcoRI-BfaI Arabidopsis fragments (i.e., fragments on which a site for the probing enzyme is either present or absent in both ecotypes). The results from these control probes allow correction for background and optical cross-talk between the two channels, as well as calibration of the red and green hybridization signals. It is anticipated that the vast majority of the processed data are unambiguous with respect to the allelic state of a sample fragment and in agreement with the gel-electrophoretic analysis. Figure 7 shows a false-color representation of the idealized results of the present experiment using a fictitious array of probes. It cannot be excluded that certain hybridization results are not in agreement with the gel-electrophoretic assay and/or that certain probes do not allow unambiguous determination of the allelic state of the cognate sample fragment. Such probes should be excluded from the micro-arrays that are used to genotype experimental Arabidopsis samples, other than the Columbia and Landsberg controls used in the present illustrative example.

In routine genotyping experiments, either one of the hybridization schemes outlined above can be used. Determination of the allelic state can be done by comparing the hybridization signals obtained with and without cleavage of the starting DNA with the probe reagent. Alternatively, allele-calling could be based on a comparison of the signals obtained with the test-sample and an appropriate control (e.g. Columbia or Landsberg DNA), both cleaved with the probe endonuclease reagent. The samples that need to be compared can, in principle, be hybridized separately but a preferred method consists of hybridizing a mixture of differentially labeled samples to the same array.

### Example 2

### Genetic Analysis in Corn

In this example, the utility of the method of the invention for marker assisted selection applications in plant and animal breeding is illustrated. Corn has been chosen because it is a typical representative of crop species having a complex genome. The large size of the genome (2,400 Mb), the frequent occurrence of repetitive DNA sequences and the high degree of genetic variation, all constitute technical challenges. In this example, an approach based on the generation of a set of genomic fragments carrying ESPs from two well-known inbred lines of corn, B73 and Mol7 from which many of the corn elite lines are derived is used. Another reason for choosing these lines is that a well-studied recombinant inbred population derived from these lines is available. This population can be used to map the set of ESPs. The genetic map of ESP markers will prove to be an effective tool for genetic selection in corn breeding. It is evident, however, ma a broader survey of the corn germplasm with a total of 10 to 20 evident, however, that a broader survey of the corn germplasm with a total of 10 to 20 lines will give a large number of additional ESPs (possibly 2 or 3 times as many) and will eventually result in a higher-resolution genetic map.

The ESP-harboring fragments could very well be identified by the gel-electrophoretic approach described for Arabidopsis (Example 1). However, an alternative strategy may be used given that the corn germplasm, like many crop species, exhibits a high degree of genetic variation. Indeed, based on previous studies, the average nucleotide sequence variation in the corn germplasm is estimated to be in the order of 1 difference in 15 to 30 nucleotides. This corresponds to a frequency in ESPs in the recognition sites of tetracutter restriction enzymes of 1 in 4. At this frequency it becomes feasible to directly examine arrays of random B73/Mol7-fragments for the presence of ESPs using the present RAA method without prior screening or selection. The strategy also lends itself readily to screening with several different probing enzymes.

In the present example, two different approaches for assaying ESPs are used. The first method (format-I RAA) is similar to the one described in Example 1, and detects ESPs in fragments sampled with a pair of restriction enzymes. In the second method (format-III RAA) individual ESPs are selectively amplified from the sampled fragments with dedicated primer sets. The principal advantage of the latter approach is that ESPs detected with several different probing enzymes can be assayed simultaneously, and that multiplex amplification of ESP-specific PCR products is made considerably more robust.

In essence the procedure described in this example comprises the following steps:
8. Identification of a set of candidate ESP fragments from the inbred lines B73 and Mol7
9. Development of a corn ESP micro-array
10. Genetic mapping of a B73/Mol7 recombinant inbred population and of segregating populations

### Step 1. Identification of candidate ESP fragments

*Cloning of a set of Sample fragments.* To clone a set of random fragments from the inbred lines B73 and Mol7, the enzyme combination PstI and BfaI is used. The hexanucleotide-recognizing enzyme PstI was chosen because of the large size of the corn genome. It is estimated that this enzyme has around 30,000 sites in the corn genome. The second tetracutter-enzyme, BfaI, is expected to cleave in the majority of the cases on both sides of the PstI sites. The double digestion will therefore generate about 60,000 sample fragments with an average size of 400-500 base pairs.

Following double digestion of the genomic DNA, PstI- and BfaI-adaptors were ligated to the fragment ends and the material amplified with non-selective PstI and BfaI primers. The structures of the PstI- and BfaI-adaptors are based on those described by Vos P. *et al. , Nucleic Acids Res.* **23**: 4407-4414 (1995): based on those described by Vos P. *et al., Nucleic Acids Res.* **23**: 44074414 (1995): The corresponding PstI and BfaI non-selective primers have the following sequences:

The amplification step enriches the PstI-BfaI fragments over the large excess of BfaI-BfaI fragments. After amplification the fragments are fractionated on an agarose gel to eliminate the fragments smaller than 100 base pair, and cloned- in an appropriate vector (e.g. TA cloning system; Invitrogen, Carlsbad, CA, U.S.A.).

*Preparation of spotted micro-arrays with the cloned sample DNA fragments.* The insert DNAs, from the two libraries of cloned PstI-BfaI sample fragments (obtained from the B73 and Mol7 inbred lines), are amplified from the clones using the non-selective PstI and BfaI primers. Following purification and concentration, the amplicons are arrayed as described in Example 1. A total of 20,000 (i.e. 10,000 from each library) candidate probe DNAs are spotted.

*Micro-array hybridization and selection of candidate ESP-fragments.* From genomic DNA of the inbred lines B73 and Mol7 four different sets of PstIlBfaI-digested amplified DNA are prepared. An alternative pair of adaptors and non-selective amplification primers are used for this: DNA has a high AT content, the preferred enzymes are those that have an AT bias in their recognition sequence. Alternatively, mixtures of two or more tetracutter or pentacutter enzymes can be used.

For each of the B73 samples, a Cy3(green)-labeled PstI primer is used, whereas the Mol7-derived fragments are amplified with a Cy5(red)-labeled PstI primer (refer to Example 1). Different hybridization solutions are then prepared by mixing equal amounts of the uncleaved, MseI-cleaved, Tsp509I-cleaved, and AluI-cleaved samples of both inbred lines. Each of the 4 mixes is allowed to hybridize to the micro-arrays. Analysis of the scanned images involved normalization using the multitude of probes on the arrays that detect monomorphic fragments. Figure 8 shows a false-color representation of the idealized results of the present experiment using a fictitious array of probes.

Analysis reveals that candidate ESP fragments are readily identified by scoring the probes that hybridize with only one of the two inbred line sample DNAs after cleavage with the probe enzyme (Figure 8). The quantitative analysis allows us the use of an unambiguous cut-off threshold of 10-fold difference in the normalized signal intensities for scoring ESPs. It should be pointed out that the assay identifies both bona fide ESPs and polymorphisms in the sampling enzyme sites. Most of the latter polymorphisms result in a marked hybridization difference with the sample DNAs not cleaved with the probe enzyme (see Figure 8). Analysis of 180 probes reveals that roughly 6% of the sample fragments carry ESPs for MseI, Tsp509I, or Alul, in accordance with the expected ESP mutation frequency. The analysis of 20,000 cloned probe fragments is thus expected to yield a total of 1,200 fragments carrying ESPs for the three probe enzymes tested. By using additional tetracutter and pentacutter enzymes (see Table I), the fraction of ESP carrying fragments may be as high as 25 %, amounting to 5,000 ESPs.

Of all probes that exhibit a differential hybridization with the cleaved sample DNAs, only those in which the recognition site for the probing enzyme is present were retained for development of a corn micro-array. Sequence determination of these probe-fragments reveals the position of the recognition site for the probe enzyme. Thus, we retained only those probes that failed to give a signal with the cleaved sample DNA from the same inbred line from which they were isolated. Such probes exhibit the hybridization pattern shown in the Table here below and are marked with an arrow in Figure 8.

| | B73/Mol7 (Cy3/Cy5) normalized hybridization signal | |
|---|---|---|
| | Undigested | MseI/Tsp509l/AIuI-digested |
| B73-probes | ⁻1 | < 0.1 |
| Mol7-probes | ⁻1 | > 10 |

### Step 2. Development of a corn ESP micro-array

*Sequencing of the candidate ESPs and design of marker specific primers.* Clones corresponding to the probes that yield the desired hybridization pattern (Figure 8) are sequenced. The majority of the insert DNAs derived from these clones contain a single recognition site for the probing enzyme. For each unique candidate ESP, two specific PCR primers, flanking the restriction site, are designed.

In addition, the sequence of a limited set of probes that yielded invariant hybridization signals is also determined. PCR primers targeting these monomorphic sequences are included as references; they are used to calibrate the hybridization signals.

Validarion *of the candidnte ESPs and fabrication of corn micro-arrays.* The candidate ESPs, identified under step 1, are subjected to a confirmatory experiment using the format-III approach. First, four pre-amplification reactions are performed with a single primer pair and using the PstI-BfaI fragments, undigested or digested with either one of the three probing enzymes, as template material. These amplification reactions reduce the complexity of the DNA under study by more than two orders of magnitude while at the same time generating a large enough amount of material for the subsequent multiplex marker-specific PCRs. The pre-amplifications are then used for the PCR rescue of each of the characterized candidate ESPs using dedicated primer couples [refer to Wang, D. G. *et al.,* Science **280**:1077-1082 (1998)]. Particular sets of the ESP-specific primers that amplify the same type of ESP (i.e. ESPs for one particular probing enzyme) are combined in a single reaction, together with the appropriate pre-amplification material as template. One of the ESP-specific primers is either Cy3- or Cy5-labeled; the other remained unlabeled. The Cy3-primers are used for the multiplex amplification of the DNA that had previously been digested with a probing enzyme, whereas the Cy5-primers are used with undigested control DNA. The PCR products from the various multiplex reactions performed on both digested and undigested DNA were pooled together to obtain a single hybridization mixture per starting DNA. The B73 and Mol7 derived material was analyzed in parallel experiments. The set of ESP-specific unlabeled PCR primers served as hybridization probes and was arrayed in the same way as amplification products. Conditions used are similar to those previously described for hybridization against oligonucleotide probes and are readily determined by one of ordinary skill in the art.

Direct comparison of the normalized Cy3 and Cy5 hybridization signals allows determination of the allelic state of the endonuclease target site in B73 versus Mol7. Primer pairs that do not allow unambiguous allele calling or that do not confirm the candidate ESPs identified with PstI-Bfal sampling (refer to step 1), are not retained for further work.

### Step 3. Genetic analysis of a B73/Mol7 recombinant inbred population and of segregating populations

*Genetic analysis of a B73*/*Mol7 inbred population.* A collection of recombinant inbred lines derived from a cross between B73 and Mol7 is publicly available and provides a most useful set of lines for verifying and mapping the collection of ESP markers. The advantage of recombinant inbred lines over segregating populations is that each inbred line contains a different set of homozygous chromosome segments derived from either parent line. Consequently each ESP will be scored as either present or absent. Preparation of the sample DNAs and hybridization against the arrayed probes are performed as described under step 2. The experiment will, in the first place, allow the testing of selected ESPs in over 100 measurements; the results will result in the development of a second generation system that will only detect the most consistent ESPs. In addition, the linkage analysis of the segregation data will allow the construction of a fine genetic map of the markers. Finally, based on the mapping data, an ordered ESP micro-array is developed for corn.

*Genetic analysis of segregating populations.* While isolated from two inbred lines, it is anticipated that the above-mentioned ordered ESP micro-arrays will detect sufficient genetic polymorphism in other corn lines to be useful for marker assisted selection. To demonstrate the applicability, one could either chose a segregating F2 population or a back-cross population. Sample preparations and hybridizations are again performed as described under step 2. In this experiment, the ESP markers must be scored quantitatively so as to differentiate between heterozygosity and homozygosity. Because only the most consistent markers are retained, a two-fold difference in signal intensity is easily monitored. The approach used consists of normalizing the hybridization signal intensities and then applying a mixture model analysis on the normalized data. This statistical approach consists of determining whether the relative signal intensities can be grouped into three discrete classes, corresponding to respectively homozygous present, heterozygous and homozygous absent. ESP markers that do not fulfill this criterion should be eliminated from the analysis.

### Example 3

### Human Genetic Analysis Using the Format-I RAA

This example illustrates the application of the method of the invention for genome-wide genetic analysis in humans. Human is an example of a high complexity genome (size ^{~}3,000 Mb) combined with a very low level of genetic variability. Single nucleotide differences between pairs of allelic sequences from different individuals occur approximately once in every 1000 basepairs; in the population at large, the frequency may be in the order of 1:300. As with Arabidopsis, such a low frequency necessitates the use of a selection procedure for the isolation/enrichment of the rare ESP-harboring fragments. In this example a batch-wise hybridization is used to accomplish this.

Based on the known mutation frequencies, it can be estimated that the ESP frequency for a tetracutter-probing enzyme is in the order of 1 in 125 recognition sites. This low level of genetic variation, in combination with the sensitivity of micro-array hybridization, limits the number of ESPs that can be detected in a single assay (typically ranging from a few hundred to one thousand, a few thousand at the most). These limitations can, to a certain extent, be overcome by choosing probing enzymes that recognize tetranucleotide sites containing a CpG dinucleotide. Indeed, it is well documented that a substantial fraction ≥25 %) of the nucleotide substitutions in the human genome result from C → T transitions in CpG dinucleotides. Such CpG dinucleotides represent mutational hotspots is vertebrates because a large fraction of the cytosines are methylated and subsequently mutate to thymine by deamination. It is estimated that the mutation frequency of methylated cytosines is 6 to 8-fold higher than average. Hence probing enzymes that cleave CpG-containing recognition sites will yield ESPs at correspondingly higher frequencies, estimated at ^{~}5 %. However, the adverse consequence of the high mutation rate is that CpG is relatively rare in mammalian DNA, occurring with a frequency of 1 in 100 nucleotides [Wang, D. G. *et al., Science* **280**:1077-1082 (1998)] instead of 1 in 16. Likewise the frequency of CpG-containing tetranucleotide sites is 1 in ^{~}1600 instead of 1 in 256 bases. To compensate for this, a probe endonuclease reagent can be used, comprising of two or more of the following complementary restriction enzymes: TaqI (TCGA), MspI (CCGG), MaeII (ACGT), and HinPI or HhaI (GCGC). It should be noted however that cleavage by MaeII as well as the isoschizomers HinPI and HhaI is blocked by methylation of the cytosine residue (C⁵) within the CpG dinucleotide. These enzymes will thus only cleave at a fraction of their sites, namely the non-methylated sites. Analysis of the large amount of publicly accessible human genomic DNA sequence shows that the cocktail of the 4 enzymes will cleave once in every 400 bp on average. The total number of sites in the genome is thus in the order of 7.5 million. Assuming that the ESP frequency is 5%, the enzyme cocktail has the potential of detecting ^{~}375,000 ESPs. In addition to using combinations of restriction endonucleases, one may also use reaction conditions that decrease the cleavage specificity. Such a strategy has been applied to obtain a restriction endonuclease reagent, designated CGaseI, that is capable of cleaving DNA at CpG dinucleotides [Mead D. *et al.,* WO 94/21663]. This CGaseI restriction endonuclease reagent may be particularly useful for the analysis of human polymorphisms using the methods of the present invention.

The example described below illustrates the approach in a limited scale assay, which characterizes the human ESPs within CpG-containing tetranucleotide recognition sites using the sampling enzyme combination PacI-BfaI. The rare cutter PacI is estimated to have only about 50,000 cleavage sites in the human genome; the frequent cutter BfaI will generate two fragments per PacI site. The enzyme combination will, therefore, create a moderately complex set of 100,000 PacI-BlaI target fragments. This fragment set captures a sizable number of CpG-containing restriction sites, estimated in the order of 40,000. Assuming a 5% ESP frequency, the number of detectable ESPs is in the order of 2000. It should be stressed that many different sampling enzyme combinations can be used and that thus a substantial fraction of the ^{~}375,000 ESPs located within NCGN-type restriction sites can be monitored.

The procedure outlined in this example comprises the following steps:
(1) Development of a set of candidate PacI-BfaI ESP fragments
(2) Genetic analysis of humans using ESP probe fragments

### Step 1. Development of a set of PacI-BfaI probe fragments

A mixture of sample fragments, derived from various individuals in the population, can be divided in three classes with respect to sites for the probing enzyme: monomorphic fragments that are devoid of a cleavage site, fragments that are always cleaved, and fragments that carry one polymorphic recognition site. Fragments that are digested will be referred to as S+ fragments and fragments lacking the site as S-fragments. Polymorphic ESP fragments will thus be the only fragments present in both the S + and S- population of sampling fragments. This forms the basis for their selection by batch-wise hybridization: only ESP fragments are capable of annealing when mixing the S + and S- fragment collections. The hybridization-selection can be performed in two different, reciprocal ways: either the S + fragments can be used to retrieve the matching S- fragments, or S- fragments are used to collect the complementary S + sampling fragments. In one approach, the selected candidate ESP fragments may be isolated by cloning, arrayed, and subsequently validated by testing various sample DNAs (e.g. the various sample DNAs used as starting material for the hybridization-selection). Candidate ESP probe fragments that appear to detect monomorphic sample fragments may either be removed from the array or retained as control elements on the array. An alternative approach consists of performing the two reciprocal hybridization-selections, cloning the selected fragments, and identification of ESPs by means of matching S+ and S- fragments. The latter strategy is outlined below.

*(i) Preparaxion of S+ and S-*_{*.*}*fragments* The preferred starting material is an equimolar mixture of genomic DNA from a number of representative individuals. Such individuals (ranging from 5 to 50) may be chosen from various CEPH (Centre d'Etude du Polymorphisme Humain) pedigrees [Wang, D. G. *et al., Science* **280**:1077-1082 (1998)]. Following cleavage of the DNA mixture with the PacI/BfaI-combination of sampling enzymes, appropriate oligonucleotide adapters as described above are ligated to the fragment ends. This template DNA is divided in two aliquots and treated separately to prepare respectively the S + and S- fragment mix. To prepare the S- fragment mix, the target DNA fragments are cleaved with the probing enzyme and then amplified. This will result in a mixture of fragments that do not contain sites for the probing enzyme. Furthermore, the S- fragment mixture may be prepared by using one biotinylated primer, such that the resulting PCR product can be captured onto a solid substrate, such as magnetic beads conjugated with streptavidin. S+ fragments are prepared by (1) amplifying the mixture of PacI-BfaI fragments, (2) digesting the PCR product with one of the four NCGN-recognizing enzymes, (3) ligating appropriate adapters to the ends generated by the probing enzyme (see EP 0 534 858, incorporated herein by reference), and (4) re-amplification of the resulting material using one primer that recognizes the probe enzyme adapter and one primer that recognizes one specific sampling enzyme adapter. Similar to the S- fragments, the amplification reaction can be performed making use of a biotinylated primer that matches the probe enzyme adaptor such that the S+ fragment mixture can be immobilized.

Two alternative pairs of PacI- and BfaI-adaptors, as well as corresponding non-selective primers are used; e.g. set I is used for the amplification of the S- fragments and set II for the preparation of S+ fragments:
Set I
Set II
The adaptor ligated to the ends generated by the NCGN-cleaving probing enzyme and the corresponding amplification primer have the following structures:

*(ii) Hybridization-selection step(s)* The S- fragment mix is hybridized to the biotinylated S+ fragments. Following hybridization, the biotinylated products are captured onto streptavidin-coated magnetic beads. The beads are repeatedly washed to remove all unhybridized fragments and thereafter the hybridized S- fragments are eluted. These are then reamplified with the PacI and BfaI primers and the hybridization-selection procedure is repeated at least once. Finally the amplified fragments are cloned in an appropriate vector and a series of around 2,000 inserts are sequenced. To select a set of S + fragments, this procedure is repeated in reverse using this time biotinylated S- fragment. Upon comparison of the S + and S- sequences ESP fragments are readily identified as fragments having partially overlapping sequences and in which the S- fragment sequence shows a mutated NCGN restriction site at the internal boundary of the overlap. In this way, ≥500 ESPs are readily characterized.

### Step 2. Genetic analysis of humans using ESP probe fragments

The sequence-verified ESP fragments are spotted on micro-arrays for genetic analysis of human sample DNA. For the preparation of this sample DNA, a pair of adaptors/primers is used that differs from those attached to the arrayed S- or S+ set of ESP fragments. From each individual, an undigested control sample and a probe enzyme digested test sample are prepared. These samples are labeled with Cy3 and Cy5, mixed and hybridized to the micro-arrays as described before. Alternatively, the hybridization mixture may be composed of differentially labeled test DNA and previously genotyped control DNA, both digested with the probing endonuclease. In both cases, the Cy3 (test/digested sample) and Cy5 (control/undigested DNA) signal intensities are normalized using a number of monomorphic control probes. The ratio of these normalized Cy3/Cy5 signals for each of the ESP probes, allows accurate determination of the allelic state of the sample at each polymorphic site (homozygous S+/S+, homozygous S-/S-, heterozygous S+/S).

The micro-array hybridization experiment may in the first place be performed with the sample DNAs, deriving from a collection of individuals, from which the ESP probe fragments were isolated. Such an experiment will, in the first place, confirm the polymorphic nature of the selected probe fragments and allow their testing in a multitude of measurements. The data will also yield information on the allele frequencies among an appreciable number of chromosomes.

### Example 4

### Human genetic analysis using format-II RAA

As described for corn in Example 2, the format-I ESP assay for human genetic analysis may be converted to a format-II or a format-III assay. Based on the sequence of the selected and experimentally validated ESP fragments, it is indeed possible to design a pair of dedicated, i.e. ESP-specific, PCR primers. Such primers can be combined in a number of parallel multiplex reactions, which are in turn combined to obtain the sample DNA [Wang, D. G. *et al., Science* **280**: 1077-1082 (1998)]. This sample DNA is hybridized against a micro-array of spotted S+ ESP fragments (*see* to Example 3). The experiment is set up such that the fluorescently labeled ESP-specific primer and the S + sequences are located on opposite sides of the polymorphic site. Alternatively, the unlabeled ESP-specific amplification primers may be arrayed as hybridization probes. The development of a format II or format-III assay need not be preceded by the identification of ESP fragments (using one of the methods described in the previous examples). In the present example, we describe the development of an RAA assay based on the sequence of previously discovered SNPs.

Close inspection of the known SNPs reveals that a significant percentage of them are associated with both the loss and gain of a restriction recognition site, i.e. each of two allelic sequences is associated with a different restriction recognition site. The single nucleotide substitution may inter-convert recognition sequences that are identical except for one nucleotide [e.g. PleI (GACTC) and HgaI (GACGC), HgaI and SfaNI (GATGC), SfaNI and Bbvl (GCTGC)]. Alternatively, the allelic recognition sites may be partially overlapping [e.g. MaeII (ACGTg) and NlaIII (aCATG); in the latter case the inter-conversion depends on the nature of the upstream or downstream sequences). Such mutually exclusive restriction site allelism offers a distinct advantage. The RAA technique will normally only detect the allele that is devoid of a recognition site for the probing enzyme; therefore, determination of the zygosity requires careful calibration of the signal against that observed with undigested control DNA. When each allele is associated with the presence/absence of a restriction site, two parallel RAA-assays can be performed, each involving digestion with one of the alternative enzymes. With such an assay, both alleles can be positively identified and the zygosity is readily determined. The two parallel assays are best performed in a two-color mode.: one of the primers is differentially labeled (e.g. with Cy3 and Cy5 as described previously) such that the amplification reactions can be mixed and hybridized against a single array of probes.

We have systematically explored the SNP database of the Whitehead Institute for mutational changes that promote restriction site inter-conversions and have calculated their occurrence frequency. Two SNP-associated recognition site inter-conversions were found to occur at high frequency: MaeII -> Main and HgaI -> SfaNI. In both cases the mutational changes converting one site into another are C→T (or G→A) transitions occurring in CpG dinucleotides. This finding is entirely consistent with the fact that this type of mutation occurs with a 6-8 times higher frequency than other nucleotide substitutions. Based on the number of SNPs found in the Whitehead database. we estimate the total number of SNPs in the human genome for the enzyme pairs MaeIIJNIaIII and HgaI/SfaNi at respectively 30,000 and 15,000. These numbers are presumably somewhat overestimated since both MaeII and Hgal are susceptible to CpG methylation. Consequently the inter-conversion can only be measured at the non-methylated sites. Therefore, in practice. RAA assays designed on the basis of sequence data should be validated on a number of test samples. Assays in which no cleavage takes place at the CpG-containing site in none of the individuals tested, should be eliminated from the RAA bi-allelic marker systems.
The foregoing examples are illustrative of the invention and are not intended to be limit the scope of the invention as set out in the claims.

## Claims

1. A method to perform multiplex genotyping whereby multiple endonuclease site polymorphisms (ESPs) are simultaneously analyzed in sample DNA, the method comprising:
(a) isolating sample DNA;
(b) treating the sample DNA of step (a) with one or more sampling restriction endonudease reagent(s) to derive a set of concomitantly amplifiable sample restriction endonudease reagent treated target DNA fragments,
(c) treating the target DNA fragments obtained in step (b) with a restriction reagent, the probe restriction endonuclease reagent; with said probe restriction endonuclease reagent being different from the sampling restriction endonuclease reagents) of step (b) to probe the allelic state at specific ESP-sites;
(d) amplifying selectively or non-selectively the probe restriction endonudease reagent treated target DNA fragments of step (c) in one or more multiplex reactions;
(e) analyzing the DNA of step (d) by hybridization to an array containing spatially addressable probes or oligonucieotides or a multitude of identifiable solid phase particles each carrying a different probe or oligonudeotide to determine which target fragments are amplified and/or which target fragments are not amplified; and wherein target DNA fragments which are amplified in step (d) lack a recognition site for the probe restriction endonuclease reagent and target fragments having a recognition site for the probe restriction endonuclease reagent are not amplified, whereby each cognate probe fragment or oligonucleotide allows to characterize one or more specific ESP(s).

2. The method of claim 1 wherein the order of the steps (b) and (c) is reversed or carried out simultaneously.

3. The method of claim 1 or 2 wherein said concomitantly amplifiable DNA fragments of step (b) are derived from sample DNA by treatment of the sample DNA with a first and a second sampling restriction endonuclease reagent.

4. The method of claim 3 wherein said first sampling restriction endonuclease reagent has a recognition sequence of six or more nucleotides and said second sampling restriction endonuclease reagent has a recognition sequence of four or fewer nucleotides.

5. The method of claim 3 or 4 wherein said concomitantly amplifiable target DNA fragments are derived by stepwise treatment of said sample DNA with the first and the second sampling restriction endonuclease reagents.

6. The method of any of claims 1 to 5 wherein said concomitantly amplifiable DNA fragments of step (b) are modified by ligation of adapters to both termini of said fragments.

7. The method of any of claims 1 to 6 wherein the DNA fragments of step (c) are pre-amplified and used as template for the amplification of step (d).

8. The method of claim 7 wherein the pre-amplification is performed using primers recognizing the adapters, ligated to the termini of all or a subset of the sampling restriction endonuclease treated DNA fragments.

9. The method of any of claims 1 to 8 wherein in step (d) the DNA fragments are amplified selectively in one or more multiplex reactions using dedicated primers that flank said ESPs, or, using primers that recognize the adapters ligated to the termini of the sampling restriction endonuclease treated DNA fragments and wherein the selective amplifications are performed with EcoRI and BfaI primers having two and three selective nucleotides recognizing the target DNA of step (b), respectively.

10. The method of any of claims 1 to 8 wherein in step (d) the DNA fragments are amplified non-selectively in a multiplex reaction using primers recognizing the adapters ligated to the termini of the sampling restriction endonuclease treated DNA fragments.

11. A method to perform multiplex genotyping whereby multiple known endonuclease site polymorphisms (ESPs) are simultaneously analyzed in sample DNA, the method comprising:
(a) isolating sample DNA;
(b) treating the sample DNA obtained in step (a) with one or more. probe restriction endonudease reagent(s) resulting in the production of probe restriction endonuclease reagent treated target DNA fragments, to probe the allelic state at specific ESP-sites,
(c) concomitantly and selectively amplifying multiple target DNA fragments having an endonuclease site polymorphism (ESP) for the probe restriction endonuclease in one or more multiplex reactions using dedicated primers that flank said ESPs;
(d) analyzing the DNA of step (c) by hybridization to an array containing spatially addressable probes or oligonucleotides or a multitude of identifiable solid phase particles each carrying a different probe or oligonucleotide to determine which target fragments are amplified and/or which target fragments are not amplified; and wherein target DNA fragments which are amplified lack a recognition site for the probe restriction endonudease reagent and target fragments having a recognition site for the probe restriction endonudease reagent are not amplified in step (c), whereby each cognate probe fragment or oligonucleotide allows to characterize one or more specific ESP(s).

12. The method of any of claims 1 to 11 wherein said probe restriction endonuclease reagent has a recognition sequence comprising six or more nucleotides.

13. The method of any of claims 1 to 11 wherein said probe restriction endonuclease reagent has a recognition sequence comprising four or more nucleotides.

14. The method of any of claims 1 to 11 wherein the said probe restriction endonuclease has a recognition sequence of two nucleotides.

15. The method of any of claims 1 to 14 wherein said endonuclease site polymorphism (ESP) is an alteration in the nucleotide sequence of a concomitantly amplifiable target fragment giving rise to a nucleotide sequence that is recognized and cut by the probe restriction endonuclease reagent.

16. The method of any of claims 1 to 14 wherein said endonuclease site polymorphism (ESP) is an alteration in the nucleotide sequence of a concomitantly amplifiable target fragment which eliminates a recognition sequence for said probe restriction endonuclease reagent.

17. The method of any of claims 1 to 16 wherein said amplification is carried out by a polymerase chain reaction.

18. The method of any of claims 1 to 17 wherein said probes for hybridization are attached to a solid support which is selected from a group consisting of a planar solid support, a bead, a sphere and a polyhedron.

19. The method of any of claims 1 to 18 wherein said DNA analyzed by hybridization consists of a mixture of two differentially labeled counterparts wherein one part is derived from the sample material with omission of the digestion with probe restriction endonuclease reagent.

20. The method of any of claims 1 to 19 wherein said hybridization probes are fragments derived from the sample DNA and having an endonuclease site polymorphism (ESP) for the probe restriction endonudease.

21. The method of any of claims 1 to 19 wherein said hybridization probes are synthetic oligonucleotides based on the sequence of concomitantly amplifiable DNA fragments having an endonudease site polymorphism (ESP) for the probe restriction endonudease.

## Patentansprüche

1. Verfahren zur Durchführung einer Multiplex-Genotypisierung, wobei eine Vielzahl von Endonuclease-Erkennungsstellen-Polymorphismen (ESPs) in einer DNA-Probe gleichzeitig analysiert werden, umfassend:
(a) Isolieren einer DNA-Probe;
(b) Behandeln der DNA-Probe aus Schritt (a) mit einem Proben-Restriktionsendonuclease-Reagens oder mehreren Proben-Restriktionsendonuclease-Reagenzien, um einen Satz von gleichzeitig amplifizierbaren, mit Proben-Restriktionsendonuclease-Reagenzien behandelten DNA-Zielfragmenten abzuleiten,
(c) Behandeln der in Schritt (b) erhaltenen DNA-Zielfragmente mit einem Restriktionsreagens, dem Sonden-Restriktionsendonuclease-Reagens; wobei das Sonden-Restriktionsendonudease-Reagens sich vom Proben-Restriktionsendonuclease-Reagens oder den Proben-Restriktionsendonuclease-Reagenzien aus Schritt (b) unterscheidet, um den allelischen Zustand an spezifischen ESP-Stellen zu untersuchen,
(d) selektives oder nicht-selektives Amplifizieren der mit Sonden-Restriktionsendonuclease-Reagens behandelten DNA-Zielfragmente aus Schritt (c) in einer oder mehreren Multiplex-Reaktionen;
(e) Analysieren der DNA aus Schritt (d) durch Hybridisierung mit einem Array, welches räumlich adressierbare Sonden oder Oligonucleotide oder eine Vielzahl von identifizierbaren Festphasenpartikeln enthält, die jeweils eine unterschiedliche Sonde oder ein unterschiedliches Oligonucleotid tragen, um zu bestimmen, welche Zielfragmente amplifiziert werden und/oder welche Zielfragmente nicht amplifiziert werden; und wobei den DNA-Zielfragmenten, welche in Schritt (d) amplifiziert werden, eine Erkennungsstelle für das Sonden-Restriktionsendonuclease-Reagens fehlt, und die Zielfragmente, die eine Erkennungsstelle für das Sonden-Restriktionsendonuclease-Reagens haben, nicht amplifiziert werden, wobei jedes verwandte Sondenfragment oder Oligonucleotid die Charakterisierung eines oder mehrerer spezifischer ESPs zulässt.

2. Verfahren nach Anspruch 1, wobei die Reihenfolge der Schritte (b) und (c) umgekehrt wird oder die Schritte gleichzeitig ausgeführt werden.

3. Verfahren nach Anspruch 1 oder 2, wobei die gleichzeitig amplifizierbaren DNA-Fragmente aus Schritt (b) aus einer DNA-Probe durch die Behandlung der DNA-Probe mit einem ersten und einem zweiten Proben-Restriktionsendonuclease-Reagens abgeleitet werden.

4. Verfahren nach Anspruch 3, wobei das erste Proben-Restriktionsendonuclease-Reagens eine Erkennungssequenz von sechs oder mehr Nucleotiden und das zweite Proben-Restriktionsendonuclease-Reagens eine Erkennungssequenz von vier oder weniger Nucleotiden hat.

5. Verfahren nach Anspruch 3 oder 4, wobei die gleichzeitig amplifizierbaren DNA-Zielfragmente durch die schrittweise Behandlung der DNA-Probe mit dem ersten und zweiten Proben-Restriktionsendonuclease-Reagens abgeleitet werden.

6. Verfahren nach einem der Ansprüche 1 bis 5, wobei die gleichzeitig amplifizierbaren DNA-Fragmente aus Schritt (b) durch die Ligierung von Adaptoren an beide Enden der Fragmente modifiziert werden.

7. Verfahren nach einem der Ansprüche 1 bis 6, wobei die DNA-Fragmente aus Schritt (c) voramplifiziert sind und als Matrize für die Amplifizierung nach Schritt (d) verwendet werden.

8. Verfahren nach Anspruch 7, wobei die Voramplifizierung durch die Verwendung von Primern ausgeführt wird, welche die Adaptoren erkennen, die an die Enden aller oder eines Teils der mit der Proben-Restriktionsendonuclease behandelten DNA-Fragmente ligiert sind.

9. Verfahren nach einem der Ansprüche 1 bis 8, wobei in Schritt (d) die DNA-Fragmente in einer oder mehreren Multiplex-Reaktionen selektiv unter Verwendung von passenden Primern, welche die ESPs flankieren, oder unter Verwendung von Primern amplifiziert werden, welche die an die Enden der mit dem Proben-Restriktionsendonuclease-Reagens behandelten DNA-Fragmente ligierten Adaptoren erkennen, und wobei die selektiven Amplifizierungen mit EcoRI- und BfaI-Primern durchgeführt werden, welche zwei bzw. drei selektive Nucleotide besitzen, die die Ziel-DNA aus Schritt (b) erkennen.

10. Verfahren nach einem der Ansprüche 1 bis 8, wobei in Schritt (d) die DNA-Fragmente in einer Multiplex-Reaktion durch die Benutzung von Primern nichtselektiv amplifiziert werden, welche die an die Enden der mit der Proben-Restriktionsendonuclease behandelten DNA-Fragmente ligierten Adaptoren erkennen.

11. Verfahren zur Durchführung einer Multiplex-Genotypisierung, wobei eine Vielzahl bekannter Endonuclease-Erkennungsstellen-Polymorphismen (ESPs) in einer DNA-Probe gleichzeitig analysiert werden, umfassend:
(a) Isolieren einer DNA-Probe;
(b) Behandeln der in Schritt (a) erhaltenen DNA-Probe mit einem Proben-Restriktionsendonuclease-Reagens oder mehreren Proben-Restriktionsendonuclease-Reagenzien, was zur Erzeugung von mit Proben-Restriktionsendonuclease-Reagens behandelten DNA-Zielfragmenten führt, um den allelischen Zustand an spezifischen ESP-Stellen zu untersuchen,
(c) gleichzeitiges und selektives Amplifizieren von mehreren DNA-Zielfragmenten, welche einen Endonuclease-Erkennungsstellen-Polymorphismus (ESP) für die Sonden-Restriktionsendonuclease in einer oder mehreren Multiplex-Reaktionen haben, unter Verwendung von passenden Primern, die die ESPs flankieren;
(d) Analysieren der DNA aus Schritt (c) durch Hybridisierung mit einem Array, welches räumlich adressierbare Sonden oder Olignucleotide oder eine Vielzahl von identifizierbaren Festphasenpartikeln enthält, die jeweils eine unterschiedliche Sonde oder ein unterschiedliches Oligonucleotid tragen, um zu bestimmen, welche Zielfragmente amplifiziert werden und/oder welche Zielfragmente nicht amplifiziert werden; und wobei den DNA-Zielfragmenten, welche amplifiziert werden, eine Erkennungsstelle für das Sonden-Restriktionsendonuclease-Reagens fehlt und die Zielfragmente, die eine Erkennungsstelle für das Sonden-Restriktionsendonuclease-Reagens haben, in Schritt (c) nicht amplifiziert werden, wobei jedes verwandte Sondenfragment oder Oligonucleotid die Charakterisierung eines oder mehrerer spezifischer ESPs zulässt.

12. Verfahren nach einem der Ansprüche 1 bis 11, wobei das Sonden-Restriktionsendonuclease-Reagens eine Erkennungssequenz hat, welche sechs oder mehr Nucleotide umfasst.

13. Verfahren nach einem der Ansprüche 1 bis 11, wobei das Sonden-Restriktionsendonuclease-Reagens eine Erkennungssequenz hat, welche vier oder mehr Nucleotide umfasst.

14. Verfahren nach einem der Ansprüche 1 bis 11, wobei die Sonden-Restriktionsendonuclease eine Erkennungssequenz von zwei Nucleotiden hat.

15. Verfahren nach einem der Ansprüche 1 bis 14, wobei der Endonuclease-Erkennungsstellen-Polymorphismus (ESP) eine Veränderung in der Nucleotidsequenz eines gleichzeitig amplifizierbaren Zielfragments ist, welche eine Nucleotidsequenz erzeugt, die von dem Sonden-Restriktionsendonuclease-Reagens erkannt und geschnitten wird.

16. Verfahren nach einem der Ansprüche 1 bis 14, wobei der Endonuclease-Erkennungsstellen-Polymorphismus (ESP) eine Veränderung in der Nucleotidsequenz eines gleichzeitig amplifizierbaren Zielfragments ist, welche eine Erkennungssequenz für das Sonden-Restriktionsendonuclease-Reagens eliminiert.

17. Verfahren nach einem der Ansprüche 1 bis 16, wobei die Amplifizierung mittels einer Polymerasekettenreaktion ausgeführt wird.

18. Verfahren nach einem der Ansprüche 1 bis 17, wobei die Sonden für die Hybridisierung an einem festen Träger befestigt sind, welcher ausgewählt ist aus der Gruppe bestehend aus einem ebenen festen Träger, einer Perle, einer Kugel und einem Polyeder.

19. Verfahren nach einem der Ansprüche 1 bis 18, wobei die durch Hybridisierung analysierte DNA aus einer Mischung von zwei unterschiedlich markierten Gegenstücken besteht, wobei ein Teil aus dem Probenmaterial unter Auslassung des Verdaus mit dem Sonden-Restriktionsendonuclease-Reagens abgeleitet ist.

20. Verfahren nach einem der Ansprüche 1 bis 19, wobei die Hybridisierungssonden Fragmente sind, welche aus der DNA-Probe abgeleitet sind und einen Endonuclease-Erkennungsstellen-Polymorphismus (ESP) für die Sonden-Restriktionsendonuclease aufweisen.

21. Verfahren nach einem der Ansprüche 1 bis 19, wobei die Hybridisierungssonden synthetische Oligonucleotide sind, welche auf der Sequenz der gleichzeitig amplifizierbaren DNA-Fragmente basieren, die einen Endonuclease-Erkennungsstellen-Polymorphismus (ESP) für die Sonden-Restriktionsendonuclease aufweist.

## Revendications

1. Procédé pour réaliser un génotypage multiplex par le biais duquel de multiples polymorphismes de sites d'endonucléase (ESP) sont analysés simultanément dans de l'ADN échantillon, le procédé comprenant :
(a) l'isolation d'un échantillon d'ADN ;
(b) le traitement de l'échantillon d'ADN de l'étape
(a) par un ou plusieurs réactifs d'échantillonnage à base d'endonucléase de restriction pour obtenir un ensemble de d'échantillons de fragments d'ADN cibles, amplifiables de manière concomitante, traités par un réactif à base d'endonucléase de restriction ;
(c) le traitement des fragments d'ADN cibles obtenus dans l'étape (b) par un réactif de restriction, le réactif sonde à base d'endonucléase de restriction, ledit réactif sonde à base d'endonucléase de restriction étant différent des réactifs d'échantillonnage à base d'endonucléase de restriction de l'étape (b) afin de sonder l'état allélique au niveau de sites ESP spécifiques ;
(d) l'amplification sélective ou non sélective des fragments d'ADN cibles traités par un réactif sonde à base d'endonucléase de restriction de l'étape (c) dans une ou plusieurs réactions multiplex ;
(e) l'analyse de l'ADN de l'étape (d) par hybridation avec une matrice contenant des sondes ou oligonucléotides spatialement adressables ou une multitude de particules identifiables de phase solide, chacune portant une sonde ou un oligonucléotide différents afin de déterminer les fragments cibles qui sont amplifiés et/ou les fragments cibles qui ne sont pas amplifiés ; et dans lequel les fragments d'ADN cibles qui sont amplifiés dans l'étape (d) sont dépourvus d'un site de reconnaissance pour le réactif sonde à base d'endonucléase de restriction et les fragments cibles, ayant un site de reconnaissance pour le réactif sonde à base d'endonucléase de restriction ne sont pas amplifiés, chaque fragment de sonde ou oligonucléotide cognatique permettant ainsi de caractériser un ou plusieurs ESP spécifiques.

2. Procédé selon la revendication 1, dans lequel l'ordre des étapes (b) et (c) est inversé ou bien elles sont réalisées simultanément.

3. Procédé selon la revendication 1 ou 2, dans lequel lesdits fragments d'ADN amplifiables de manière concomitante de l'étape (b) sont dérivés de l'ADN échantillon en traitant l'ADN échantillon par un premier et un deuxième réactifs d'échantillonnage à base d'endonucléase de restriction.

4. Procédé selon la revendication 3, dans lequel ledit premier réactif d'échantillonnage à base d'endonucléase de restriction possède une séquence de reconnaissance de six nucléotides ou plus et ledit deuxième réactif d'échantillonnage à base d'endonucléase de restriction possède une séquence de reconnaissance de quatre nucléotides ou moins.

5. Procédé selon la revendication 3 ou 4, dans lequel lesdits fragments d'ADN cibles amplifiables de manière concomitante sont obtenus à l'aide d'un traitement par étapes dudit ADN échantillon par les premier et deuxième réactifs d'échantillonnage à base d'endonucléase de restriction.

6. Procédé selon l'une quelconque des revendications 1 à 5, dans lequel lesdits fragments d'ADN amplifiables de manière concomitante de l'étape (b) sont modifiés par ligature d'adaptateurs aux deux terminaisons desdits fragments.

7. Procédé selon l'une quelconque des revendications 1 à 6, dans lequel les fragments d'ADN de l'étape (c) sont pré-amplifiés et employés en tant que matrice pour l'amplification de l'étape (d).

8. Procédé selon la revendication 7, dans lequel la pré-amplification se réalise en employant des amorces qui reconnaissent les adaptateurs, ligaturées aux terminaisons de la totalité ou d'un sous-ensemble des fragments d'ADN traités par l'endonucléase de restriction d'échantillonnage.

9. Procédé selon l'une quelconque des revendications 1 à 8, dans lequel dans l'étape (d) les fragments d'ADN sont amplifiés sélectivement dans une ou plusieurs réactions multiplex employant des amorces dédiées qui flanquent lesdits ESP, ou employant des amorces qui reconnaissent les adaptateurs, ligaturées aux terminaisons des fragments d'ADN traités par l'endonucléase de restriction d'échantillonnage et dans lequel les amplifications sélectives sont réalisées avec des amorces EcoRI et BfaI ayant respectivement deux et trois nucléotides sélectifs reconnaissant l'ADN cible de l'étape (b).

10. Procédé selon l'une quelconque des revendications 1 à 8, dans lequel dans l'étape (d) les fragments d'ADN sont amplifiés de manière non sélective dans une réaction multiplex en employant des amorces reconnaissant les adaptateurs, ligaturées aux terminaisons des fragments d'ADN traités par l'endonucléase de restriction d'échantillonnage.

11. Procédé pour réaliser un génotypage multiplex par le biais duquel des polymorphismes multiples connus de sites d'endonucléase (ESP) sont analysés simultanément dans de l'ADN échantillon, le procédé comprenant :
(a) l'isolement de l'ADN échantillon ;
(b) le traitement de l'ADN échantillon obtenu dans l'étape (a) par un ou plusieurs réactifs sondes à base d'endonucléase de restriction, aboutissant à la production de fragments d'ADN cibles traités par réactif sonde à base d'endonucléase de restriction, afin de sonder l'état allélique à des sites ESP spécifiques ;
(c) l'amplification concomitante et sélective de multiples fragments d'ADN cibles ayant un polymorphisme de site d'endonucléase (ESP) pour la sonde à base d'endonucléase de restriction dans une ou plusieurs réactions multiplex employant des amorces dédiées qui flanquent lesdits ESP ;
(d) l'analyse de l'ADN de l'étape (c) par hybridation en une matrice contenant des sondes ou oligonucléotides spatialement adressables ou une multitude de particules identifiables de phase solide, chacune portant une sonde ou un oligonucléotide différents afin de déterminer les fragments cibles qui sont amplifiés et/ou les fragments cibles qui ne sont pas amplifiés ; et dans lequel les fragments d'ADN cibles qui sont amplifiés, sont dépourvus d'un site de reconnaissance pour le réactif sonde à base d'endonucléase de restriction et les fragments cibles ayant un site de reconnaissance pour le réactif sonde à base d'endonucléase de restriction ne sont pas amplifiés dans l'étape (c), chaque fragment ou oligonucléotide de sonde cognatique permettant ainsi de caractériser un ou plusieurs ESP spécifique.

12. Procédé selon l'une quelconque des revendications 1 à 11, dans lequel ledit réactif sonde à base d'endonucléase de restriction possède une séquence de reconnaissance comprenant six nucléotides ou plus.

13. Procédé selon l'une quelconque des revendications 1 à 11, dans lequel ledit réactif sonde à base d'endonucléase de restriction possède une séquence de reconnaissance comprenant quatre nucléotides ou plus.

14. Procédé selon l'une quelconque des revendications 1 à 11, dans lequel ladite sonde à base d'endonucléase de restriction possède une séquence de reconnaissance de deux nucléotides.

15. Procédé selon l'une quelconque des revendications 1 à 14, dans lequel ledit polymorphisme de site d'endonucléase (ESP) est une modification de la séquence des nucléotides d'un fragment cible amplifiable de manière concomitante donnant naissance à une séquence de nucléotides qui est reconnue et coupée par le réactif sonde à base d'endonucléase de restriction.

16. Procédé selon l'une quelconque des revendications 1 à 14, dans lequel ledit polymorphisme de site d'endonucléase (ESP) est une modification de la séquence des nucléotides d'un fragment cible amplifiable de manière concomitante qui élimine une séquence de reconnaissance pour ledit réactif sonde à base d'endonucléase de restriction.

17. Procédé selon l'une quelconque des revendications 1 à 16, dans lequel ladite amplification est réalisée à l'aide d'une réaction en chaîne par polymérase.

18. Procédé selon l'une quelconque des revendications 1 à 17, dans lequel lesdites sondes pour l'hybridation sont fixées à un support solide qui est choisi dans le groupe formé par un support solide plan, une bille, une sphère et un polyèdre:

19. Procédé selon l'une quelconque des revendications 1 à 18, dans lequel ledit ADN analysé par hybridation se compose d'un mélange de deux contreparties marquées de façon différente dans lesquelles une partie provient de la matière échantillon avec omission de la digestion par le réactif sonde à base d'endonucléase de restriction.

20. Procédé selon l'une quelconque des revendications 1 à 19, dans lequel lesdites sondes d'hybridation sont des fragments provenant de l'ADN échantillon et possédant un polymorphisme de site d'endonucléase (ESP) pour la sonde à base d'endonucléase de restriction.

21. Procédé selon l'une quelconque des revendications 1 à 19, dans lequel lesdites sondes d'hybridation sont des oligonucléotides de synthèse basés sur la séquence de fragments d'ADN amplifiables de manière concomitante possédant un polymorphisme de site d'endonucléase (ESP) pour la sonde à base d'endonucléase de restriction.
